# EUROPEAN PATENT APPLICATION

(11) **EP 4 806 457 A1**
(43) Date of publication of application: **16.09.2026**
(21) Application number: 24888027.0
(22) Date of filing: 06.11.2024
(51) Int. Cl.: A61K 38/26, A61P 3/04

(54) **GLP-1 COMPOUND FOR WEIGHT LOSS**

(30) Priority: 06.11.2023 CN 202311464189; 02.06.2024 CN 202410702374; 22.07.2024 CN 202410984951; 02.08.2024 CN 202411055430; 30.10.2024 CN 202411531954
(71) Applicant: GAN & LEE PHARMACEUTICALS CO., LTD., Beijing 101109 (CN)
(72) Inventor: CHEN, Wei, Beijing 101109 (CN); GAN, Zhongru, Beijing 101109 (CN); ZHAO, Liyuan, Beijing 101109 (CN)
(74) Representative: Kraus & Lederer PartGmbB
(86) International application number: PCT/CN2024/130351
(87) International publication number: WO 2025/098416

(57) **Abstract**

The present invention relates to a GLP-1 compound for weight loss.

## Description

### TECHNICAL FIELD

The present invention relates to the field of biopharmaceuticals and relates to GLP-1 compounds for treating obesity.

### BACKGROUND

Obesity is a condition characterized by excessive fat accumulation in the body that leads to health impairment. Over the past two decades, obesity has become a serious global health and social issue. In a study involving over 192 million people, researchers analyzed global data trends from 1975 to 2014 and projected that by 2025, the global obesity rate will reach 18% for men while the female proportion will exceed 21%. Among these, 6% of men and 9% of women will have a Body Mass Index (BMI) exceeding 40 kg/m². In China, surveys indicate 266 million adults are overweight and 53 million are obese, representing 27.8% and 5.5% of the adult population respectively (Yang Wenying, Lu Juming, Weng Jiangping, et al. Prevalence of diabetes among men and women in China [J]. N Engl J Med. 2010, 362(25):2425-2426.).

In 2017, obesity was classified by the World Obesity Federation as a chronic, recurrent disease, and recognized as the result of interactions between genetic, metabolic, environmental, and behavioral factors. Obesity also increases morbidity and mortality rates. Compared to individuals of normal weight, obese individuals are more susceptible to coronary heart disease, hypertension, hyperlipidemia, diabetes mellitus, cerebrovascular accidents, osteoarthritis, obstructive sleep apnea, and other diseases. Some studies also indicate obesity is associated with higher incidence rates of certain cancers. Compared to age-matched individuals of normal weight, obese individuals face increased mortality risk from all causes.

Traditionally, lifestyle interventions such as dietary counseling, behavioral therapy, and exercise guidance have been the primary treatment approaches for obesity. However, the causes of obesity are multifactorial, involving numerous complex environmental, physiological, and genetic factors, which also make its treatment challenging. Scientific evidence indicates that weight gain triggers hormonal, metabolic, and neurochemical adaptations that may disrupt energy balance regulation, promote weight gain maintenance, and make weight loss difficult. Furthermore, during weight loss, the body appears to compensate by reducing metabolism and increasing the production of appetite-stimulating hormones. These combined effects often favor weight regain and explain why sustained weight loss is difficult to achieve and why many people struggle to maintain their weight through lifestyle interventions alone.

Surgical treatment offers an effective alternative for some severely obese patients. However, these methods are unavailable or unsuitable for many obese patients and are often associated with risks and complications inherent to bariatric surgery. In one study, the incidence rates of major and minor complications were 3.3% and 27%, respectively.

Therefore, few effective treatment options exist for obese patients, particularly those with obesity-related health issues. Pharmacological treatment can serve as a valuable adjunct to lifestyle interventions for achieving and maintaining weight loss; it may also reduce metabolic responses that promote weight regain. However, currently available weight-loss medications are extremely limited.

Efforts to treat obesity with drugs have been plagued by safety concerns, leading to some drugs never receiving approval in the United States (rimonabant), or withdrawn from the U.S. market after approval due to severe toxicity (fenfluramine, dexfenfluramine, phentolamine, and sibutramine). In recent years, new drugs have received approval. To date, drugs approved by the U.S. Food and Drug Administration (FDA) for treating chronic obesity include: Orlistat, Xenical, Phentermine, Naltrexone, Phenbutazone/Tolbutamide, Liraglutide, and Semaglutide. Researchers have now discovered that GLP-1 analogues or GLP-1 derivatives may possess weight-loss potential. The present invention provides a GLP-1 derivative weight-loss pharmaceutical with excellent properties, along with appropriate dosages and administration frequencies.

### SUMMARY

The present disclosure is related to a method for treating obesity, which comprises administering to a subject in need a therapeutically effective amount of a compound (I), wherein the subject has clinical evidence of obesity; wherein the therapeutic effect of the method is superior to that of 2.4 mg of semaglutide injection and 15 mg of tirzzepatide, and can reduce the occurrence of obesity-related adverse events (MACE).

The present disclosure provides a method for treating or preventing obesity, wherein the method comprises administering to a subject in need thereof about 0.25 mg to about 100 mg of a compound of formula (I), or apharmaceutically acceptable salt, amide, or ester thereof,

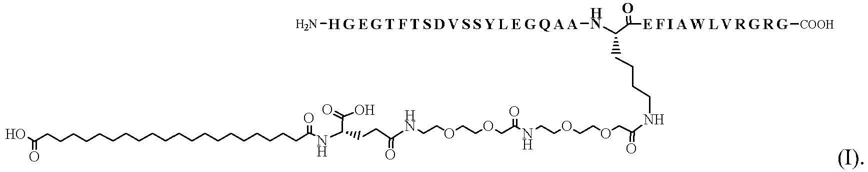

In some embodiments, wherein the method comprises administering to a subject in need thereof about 0.25-100 mg, preferably about 0.5-90 mg, preferably about 1-80 mg, preferably about 1.5-80 mg, preferably about 2-80 mg, preferably about 1.5-70 mg, preferably about 2-70 mg, preferably about 3-70 mg, preferably about 1.5-60 mg, preferably about 3-60 mg, preferably about 1.5-55 mg, preferably about 3-55 mg, preferably about 1.5-48 mg, preferably about 3-48 mg, preferably about 1.5-36 mg, preferably about 3-36 mg, preferably about 1.5-30 mg, preferably about 3-30 mg, preferably about 1.5-24 mg, preferably about 3-24 mg, preferably about 1.5-18 mg, preferably about 3-18 mg, preferably about 1.5-12 mg, preferably about 3-12 mg, preferably about 4-60 mg, preferably about 5-60 mg, preferably about 7-60 mg, preferably about 8-60 mg, preferably about 9-60 mg, preferably about 10-60 mg, preferably about 11-60 mg, preferably about 12-60 mg, preferably about 6-48 mg, preferably about 7-48 mg, preferably about 9-48 mg, preferably about 12-48 mg, preferably about 15-48 mg, or preferably about 24-48 mg of the compound of formula (I), or the pharmaceutically acceptable salt, amide, or ester thereof.

In some embodiments, wherein the method comprises administering to a subject in need thereof the compound of formula (I), or the pharmaceutically acceptable salt, amide, or ester thereof, at a weekly or biweekly dose of about 0.25-100 mg, preferably about 0.5-90 mg, preferably about 1-80 mg, preferably about 1.5-80 mg, preferably about 2-80 mg, preferably about 1.5-70 mg, preferably about 2-70 mg, preferably about 3-70 mg, preferably about 3-60 mg, preferably about 1.5-55 mg, preferably about 3-55 mg, preferably about 1.5-48 mg, preferably about 3-48 mg, preferably about 1.5-36 mg, preferably about 3-36 mg, preferably about 1.5-30 mg, preferably about 3-30 mg, preferably about 1.5-24 mg, preferably about 3-24 mg, preferably about 1.5-18 mg, preferably about 3-18 mg, preferably about 1.5-12 mg, preferably about 3-12 mg, preferably about 4-60 mg, preferably about 5-60 mg, preferably about 7-60 mg, preferably about 8-60 mg, preferably about 9-60 mg, preferably about 10-60 mg, preferably about 11-60 mg, preferably about 12-60 mg, preferably about 6-48 mg, preferably about 7-48 mg, preferably about 9-48 mg, preferably about 12-48 mg, preferably about 15-48 mg, preferably about 24-48 mg.

In some embodiments, wherein the method comprises administering to a subject in need thereof the compound of formula (I), or the pharmaceutically acceptable salt, amide, or ester thereof, at a weekly or biweekly dose of about 0.25 mg, about 0.5 mg, about 1 mg, about 1.5 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, or about 70 mg.

In some embodiments, wherein the method comprises administering to a subject in need thereof the compound of formula (I), or the pharmaceutically acceptable salt, amide, or ester thereof, once weekly or less frequently, preferably once every two weeks or less frequently.

In some embodiments, wherein the method comprises administering to a subject in need thereof the compound of formula (I), or the pharmaceutically acceptable salt, amide, or ester thereof, at a frequency of once weekly or once every two weeks, at the following dose: about 0.25 mg, about 0.5 mg, about 1 mg, about 1.5 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, or about 70 mg.

In some embodiments, wherein the method comprises first administering an escalation dose to a subject in need thereof at a frequency of once weekly or once every two weeks during an escalation dose period, and subsequently administering a maintenance dose at a frequency of once weekly or once every two weeks during a maintenance dose period,
wherein the escalation dose period is at least two weeks, preferably about 2 to 60 weeks, more preferably about 2 to 55 weeks, more preferably about 2 to 50 weeks, more preferably about 2 to 40 weeks, more preferably about 2 to 31 weeks, more preferably about 2 to 28 weeks, more preferably about 2 to 30 weeks, more preferably about 2 to 20 weeks, and preferably the escalation dose period is about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, about 52 weeks, about 53 weeks, about 54 weeks, about 55 weeks, about 56 weeks, about 57 weeks, about 58 weeks, about 59 weeks, or about 60 weeks;
the maintenance dose period is at least about two weeks, preferably about 3 weeks or more, about 4 weeks or more, about 5 weeks or more, about 6 weeks or more, about 7 weeks or more, about 8 weeks or more, about 9 weeks or more, about 10 weeks or more, about 11 weeks or more, about 12 weeks or more or more, about 13 weeks, about 14 weeks or more, about 15 weeks or more, about 16 weeks or more, about 17 weeks or more, about 18 weeks or more, about 19 weeks or more, about 20 weeks or more, about 21 weeks or more, about 22 weeks or more, about 23 weeks or more, about 24 weeks or more, about 25 weeks or more, about 26 weeks or more, about 27 weeks or more, about 28 weeks or more, about 29 weeks or more, about 30 weeks or more, about 31 weeks or more, about 32 weeks or more, about 33 weeks or more, about 34 weeks or more, about 35 weeks or more, about 36 weeks or more, about 37 weeks or more, about 38 weeks or more, about 39 weeks or more, about 40 weeks or more, about 41 weeks or more, about 42 weeks or more, about 43 weeks or more, about 44 weeks or more, about 45 weeks or more, about 46 weeks or more, about 47 weeks or more, about 48 weeks or more, about 49 weeks or more, about 50 weeks or more, about 51 weeks or more, about 52 weeks or more, about 53 weeks or more, about 54 weeks or more, about 55 weeks or more, about 56 weeks or more, about 57 weeks or more, about 58 weeks or more, about 59 weeks or more, or about 60 weeks or more; preferably about 2-120 weeks, preferably about 2-100 weeks, preferably about 2-80 weeks, preferably about 2-70 weeks, preferably about 2-60 weeks, preferably about 2-55 weeks, preferably about 2-50 weeks, preferably about 2-40 weeks, preferably about 2-35 weeks, preferably about 2-30 weeks, preferably about 2-20 weeks; preferably the maintenance dosage period is about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, about 52 weeks, about 53 weeks, about 54 weeks, about 55 weeks, about 56 weeks, about 57 weeks, about 58 weeks, about 59 weeks, or about 60 weeks;
the escalation dose is independently about 0.25mg-70mg of the compound of formula (I), or the pharmaceutically acceptable salt, amide or ester thereof per administration; preferably, the escalation dose is independently about 0.25 mg, about 0.5 mg, about 1 mg, about 1.5 mg, about 2 mg, about 3 mg, about 4 mg, about 5 mg, about 6 mg, about 7 mg, about 7.5 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, or about 70 mg of the compound of formula (I), or the pharmaceutically acceptable salt, amide, or ester thereof per administration;
the maintenance dose is about 3-70 mg of the compound of formula (I), or the pharmaceutically acceptable salt, amide, or ester thereof; preferably, the maintenance dosage is about 3 mg, about 4 mg, about 5 mg, about 6 mg, about 7 mg, about 7.5 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, or about 70 mg of the compound of formula (I), or the pharmaceutically acceptable salt, amide, or ester thereof; and
the maintenance dose is greater than or equal to the escalation dose;
preferably:
   during the escalation dose period, the administration period for each different escalation dose is independently about 1 week or more, about 2 weeks or more, about 3 weeks or more, about 4 weeks or more, about 5 weeks or more, about 6 weeks or more, about 7 weeks or more, about 8 weeks or more, about 9 weeks or more, about 10 weeks or more, about 11 weeks, about 12 weeks or more, about 13 weeks or more, about 14 weeks or more, about 15 weeks or more, about 16 weeks or more, about 17 weeks or more, about 18 weeks, about 19 weeks or more, or about 20 weeks or more; preferably, during the escalation dose period, the administration period for each different escalation dose is independently about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, or about 20 weeks;
preferably:
   during the escalation dose period, the escalation dose is about 1.1-3 times the preceding different escalation dose, preferably about 1.1-2 times, more preferably about 1.1 times, about 1.2 times, about 1.3 times, about 1.4 times, about 1.5 times, about 1.6 times, about 1.7 times, about 1.8 times, about 1.9 times, about 2.1 times, about 2.2 times, about 2.3 times, about 2.4 times, about 2.5 times, about 2.6 times, about 2.7 times, about 2.8 times, about 2.9 times, or about 3 times.

In some embodiments, wherein the escalation dose period is about 8-31 weeks, preferably about 10-31 weeks, preferably the escalation dose period is about 10 weeks, about 12 weeks, about 14 weeks, about 16 weeks, about 18 weeks, about 20 weeks, about 26 weeks, about 27 weeks, about 28 weeks, about 30 weeks or about 31 weeks; preferably the escalation dose period is about 10 weeks, about 14 weeks, about 18 weeks, about 28 weeks, or about 31 weeks;
the maintenance dose period is about 4 weeks or more, 5 weeks or more, about 12 weeks or more, about 14 weeks or more, about 16 weeks or more, about 18 weeks or more, about 20 weeks or more, about 30 weeks or more, about 40 weeks or more, about 50 weeks or more, about 60 weeks or more, about 70 weeks or more, about 80 weeks or more, or about 90 weeks or more; preferably the maintenance dose period is about 4-20 weeks, preferably the maintenance dose period is about 4 weeks, about 5 weeks, about 12 weeks, about 14 weeks, about 16 weeks, about 18 weeks, or about 20 weeks;
the escalation dose is independently about 1.5mg, about 3mg, about 5mg, about 6mg, about 7mg, about 7.5mg, about 9mg, about 12mg, about 15mg, about 18mg, about 24mg, about 30mg, about 36mg, or about 48mg of the compound of formula (I), or the pharmaceutically acceptable salt, amide, or ester thereof per administration; preferably, the escalation dose is independently about 3mg, about 6mg, about 9mg, about 12mg, about 15mg, about 18mg, about 24mg, or about 36mg of the compound of formula (I), or the pharmaceutically acceptable salt, amide, or ester thereof per administration; or the escalation dose is independently about 1.5mg, about 3mg, about 5mg, about 7mg, about 9mg, about 12mg, about 15mg, about 18mg, or about 24mg of the compound of formula (I), or the pharmaceutically acceptable salt, amide, or ester thereof per administration; or the escalation dose is independently about 3mg, about 6mg, about 9mg, about 12mg, about 18mg, about 24mg, about 36mg or 48mg of the compound of formula (I), or the pharmaceutically acceptable salt, amide or ester thereof per administration;
the maintenance dose is about 5mg, about 12mg, about 18mg, about 24mg, about 30mg, about 36mg, or about 48mg of the compound of formula (I), or the pharmaceutically acceptable salt, amide, or ester thereof; and
the maintenance dose is greater than or equal to the escalation dose.

9. The method according to claim 7 or 8, wherein the method comprises first administering the escalation dose to a subject in need thereof at a frequency of once weekly or once every two weeks during the escalation dose period, and then administering the maintenance dose at a frequency of once weekly or once every two weeks during the maintenance dose period, wherein,
the escalation dose period is about 10 weeks, about 14 weeks, about 18 weeks, about 28 weeks, about 30 weeks or about 31 weeks;
the maintenance dose period is about 4 weeks or more, about 12 weeks or more, about 16 weeks or more, or about 20 weeks or more; preferably, the maintenance dose period is about 4 weeks, about 12 weeks, about 16 weeks, or about 20 weeks;
the escalation dose is independently about 1.5mg, about 3mg, about 5mg, about 6mg, about 7mg, about 9mg, about 12mg, about 15mg, about 18mg, about 24mg, about 30mg, about 36mg or about 48mg of the compound of formula (I), or the pharmaceutically acceptable salt, amide or ester thereof per administration;
the maintenance dose is about 5mg, about 12mg, about 18mg, about 24mg, about 30mg, about 36mg, or about 48mg of the compound of formula (I), or the pharmaceutically acceptable salt, amide, or ester thereof; and
the maintenance dose is greater than or equal to the escalation dose;
preferably:
   when the maintenance dose is about 12mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide, or ester thereof at a frequency of once every two weeks for about 2 or more or 4 weeks or more; administering about 6mg of the compound of formula (I), or the pharmaceutically acceptable salt, amide or ester thereof at a frequency of once every two weeks for about 4 weeks or more; administering about 9mg of the compound of formula (I), or the pharmaceutically acceptable salt, amide or ester thereof at a frequency of once every two weeks for about 4 weeks or more; and administering about 12mg of the compound of formula (I), or the pharmaceutically acceptable salt, amide or ester thereof at a frequency of once every two weeks for 20 weeks or more; preferably for 25 weeks or more, preferably for 30 weeks or more, preferably for about 20-120 weeks, preferably for about 20-100 weeks, preferably for about 20-80 weeks; preferably for about 20-60 weeks; or
   when the maintenance dose is about 18mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once every two weeks for about 2 weeks or more or 4 weeks or more; administering about 6mg of the compound of formula (I), or the pharmaceutically acceptable salt, amide or ester thereof at a frequency of once every two weeks for about 4 weeks or more; administering about 9mg of the compound of formula (I), or the pharmaceutically acceptable salt, amide or ester thereof at a frequency of once every two weeks for about 4 weeks or more, administering about 12mg of the compound of formula (I), or the pharmaceutically acceptable salt, amide or ester thereof at a frequency of once every two weeks for about 4 weeks or more; and administering about 18mg of the compound of formula (I), or the pharmaceutically acceptable salt, amide or ester thereof at a frequency of once every two weeks for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
   when the maintenance dose is about 24mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once every two weeks for about 2 weeks or more or 4 weeks or more; administering about 6mg of the compound of formula (I), or the pharmaceutically acceptable salt, amide or ester thereof at a frequency of once every two weeks for about 4 weeks or more; administering about 9mg of the compound of formula (I), or the pharmaceutically acceptable salt, amide or ester thereof at a frequency of once every two weeks for about 4 weeks or more administering about 12mg of the compound of formula (I), or the pharmaceutically acceptable salt, amide or ester thereof at a frequency of once every two weeks for about 4 weeks or more; administering about 18mg of the compound of formula (I), or the pharmaceutically acceptable salt, amide or ester thereof at a frequency of once every two weeks for about 4 weeks or more; administering about 24mg of the compound of formula (I), or the pharmaceutically acceptable salt, amide or ester thereof at a frequency of once every two weeks for 12 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
   when the maintenance dose is about 48mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once every two weeks for about 2 or more or 4 weeks or more; administering about 6mg of the compound of formula (I), or the pharmaceutically acceptable salt, amide or ester thereof at a frequency of once every two weeks for about 4 weeks or more; administering about 12mg of the compound of formula (I), or the pharmaceutically acceptable salt, amide or ester thereof at a frequency of once every two weeks for about 4 weeks or more; administering about 24mg of the compound of formula (I), or the pharmaceutically acceptable salt, amide or ester thereof at a frequency of once every two weeks for about 4 weeks or more; administering about 36mg of the compound of formula (I), or the pharmaceutically acceptable salt, amide or ester thereof at a frequency of once every two weeks for about 4 weeks or more; administering about 48mg of the compound of formula (I), or the pharmaceutically acceptable salt, amide or ester thereof at a frequency of once every two weeks for 12 weeks or more, preferably for 14 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
   when the maintenance dose is about 48mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once every two weeks for about 2 weeks or more or 4 weeks or more; administering about 6mg of the compound of formula (I), or the pharmaceutically acceptable salt, amide or ester thereof at a frequency of once every two weeks for about 4 weeks or more; administering about 9mg of the compound of formula (I), or the pharmaceutically acceptable salt, amide or ester thereof at a frequency of once every two weeks for about 4 weeks or more; administering about 12mg of the compound of formula (I), or the pharmaceutically acceptable salt, amide or ester thereof at a frequency of once every two weeks for about 4 weeks or more; administering about 18mg of the compound of formula (I), or the pharmaceutically acceptable salt, amide or ester thereof at a frequency of once every two weeks for about 4 weeks or more; administering about 24mg of the compound of formula (I), or the pharmaceutically acceptable salt, amide or ester thereof at a frequency of once every two weeks for about 4 weeks or more; administering about 36mg of the compound of formula (I), or the pharmaceutically acceptable salt, amide or ester thereof at a frequency of once every two weeks for about 4 weeks or more; administering about 48mg of the compound of formula (I), or the pharmaceutically acceptable salt, amide or ester thereof at a frequency of once every two weeks for 12 weeks or more, preferably for 14 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
   when the maintenance dose is about 36mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once every two weeks for about 2 weeks or more or 4 weeks or more; administering about 6mg of the compound of formula (I), or the pharmaceutically acceptable salt, amide or ester thereof at a frequency of once every two weeks for about 4 weeks or more; administering about 12mg of the compound of formula (I), or the pharmaceutically acceptable salt, amide or ester thereof at a frequency of once every two weeks for about 4 weeks or more; administering about 18mg of the compound of formula (I), or the pharmaceutically acceptable salt, amide or ester thereof at a frequency of once every two weeks for about 4 weeks or more; administering about 24mg of the compound of formula (I), or the pharmaceutically acceptable salt, amide or ester thereof at a frequency of once every two weeks for about 4 weeks or more; and administering about 36mg of the compound of formula (I), or the pharmaceutically acceptable salt, amide or ester thereof at a frequency of once every two weeks for 12 weeks or more, preferably for 14 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
   when the maintenance dose is about 24mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once weekly for about 2 weeks or more or 4 weeks or more; administering about 6mg of the compound of formula (I), or the pharmaceutically acceptable salt, amide or ester thereof once weekly for 4 weeks or more; administering about 9mg of the compound of formula (I), or the pharmaceutically acceptable salt, amide or ester thereof at a frequency of once weekly for 4 weeks or more; administering about 12mg of the compound of formula (I), or the pharmaceutically acceptable salt, amide or ester thereof at a frequency of once weekly for 4 weeks or more; administering about 15mg of the compound of formula (I), or the pharmaceutically acceptable salt, amide or ester thereof at a frequency of once weekly for 4 weeks or more; and administering about 24mg of the compound of formula (I), or the pharmaceutically acceptable salt, amide or ester thereof at a frequency of once weekly for 12 weeks or more, preferably for 14 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
   when the maintenance dose is about 30mg of the compound of formula (I), or the pharmaceutically acceptable salt, amide or ester thereof, the method comprises sequentially administering to a subject in need thereof about 1.5mg of the compound of formula (I), or the pharmaceutically acceptable salt, amide or ester thereof at a frequency of once weekly for about 1 week or more; administering about 3mg of the compound of formula (I), or the pharmaceutically acceptable salt, amide or ester thereof at a frequency of once weekly for 2 weeks or more; administering about 5mg of the compound of formula (I), or the pharmaceutically acceptable salt, amide or ester thereof at a frequency of once weekly for 4 weeks or more; administering about 7mg of the compound of formula (I), or the pharmaceutically acceptable salt, amide or ester thereof at a frequency of once weekly for 4 weeks or more; administering about 9mg of the compound of formula (I), or the pharmaceutically acceptable salt, amide or ester thereof at a frequency of once weekly or once every two weeks for 4 weeks or more; administering about 12mg of the compound of formula (I), or the pharmaceutically acceptable salt, amide or ester thereof at a frequency of once weekly or once every two weeks for 4 weeks or more; administering about 15mg of the compound of formula (I), or the pharmaceutically acceptable salt, amide or ester thereof at a frequency of once weekly or once every two weeks for 4 weeks or more; administering about 18mg of the compound of formula (I), or the pharmaceutically acceptable salt, amide or ester thereof at a frequency of once weekly or once every two weeks for 4 weeks or more; administering about 24mg of the compound of formula (I), or the pharmaceutically acceptable salt, amide or ester thereof at a frequency of once weekly or once every two weeks for about 4 weeks or more; and administering about 30mg of a compound of formula (I), or the pharmaceutically acceptable salt, amide or ester thereof at a frequency of once weekly or once every two weeks for 4 weeks or more, preferably for 14 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks.

In some embodiments, wherein the method comprises administering to a subject in need thereof a first dose of about 0.25-5mg of the compound of formula (I), or the pharmaceutically acceptable salt, amide or ester thereof at a frequency of once weekly or once every two weeks;
after administering the first dose for at least about 1 week, preferably about 1, about 2, about 3, about 4, about 5, about 6, about 7, or about 8 weeks, administering a second dose of about 3-12mg of the compound of formula (I), or the pharmaceutically acceptable salt, amide, or ester thereof at a frequency of once weekly or once every two weeks; preferably, after administering the first dose for about 1, about 2, about 3 or about 4 weeks, administering the second dose of about 3-12mg of the compound of formula (I), or the pharmaceutically acceptable salt, amide or ester thereof at a frequency of once weekly or once every two weeks;
after administering the second dose for about 2, about 3, about 4, about 5, about 6, about 7, or about 8 weeks, administering a third dose of about 6-24mg of the compound of formula (I), or the pharmaceutically acceptable salt, amide, or ester thereof at a frequency of once weekly or once every two weeks; and
after administering the third dose for about 3, about 4, about 5, about 6, about 7, or about 8 weeks, administering a fourth dose of about 9-36mg of the compound of formula (I), or the pharmaceutically acceptable salt, amide, or ester thereof at a frequency of once weekly or once every two weeks.

In some embodiments, wherein after administering the fourth dose for about 3, about 4, about 5, about 6, about 7, about 8, about 9, about 10, about 11, about 12, about 13, about 14, about 15, about 16, about 17, about 18, about 19, or about 20 weeks, administering a fifth dose of about 12-48mg of the compound of formula (I) or the pharmaceutically acceptable salt, amide, or ester thereof at a frequency of once weekly or once every two weeks.

In some embodiments, wherein after administering the fifth dose for about 3, about 4, about 5, about 6, about 7, about 8, about 9, about 10, about 11, about 12, about 13, about 14, about 15, about 16, about 17, about 18, about 19, or about 20 weeks, administering a sixth dose of about 18-60mg of the compound of formula (I) or the pharmaceutically acceptable salt, amide, or ester thereof at a frequency of once weekly or once every two weeks.

In some embodiments, wherein after administering the sixth dose for about 2, about 3, about 4, about 5, about 6, about 7, about 8, about 9, about 10, about 11, about 12, about 13, about 14, about 15, about 16, about 17, about 18, about 19, or about 20 weeks, administering a seventh dose of about 24-72mg of the compound of formula (I), or the pharmaceutically acceptable salt, amide, or ester thereof at a frequency of once weekly or once every two weeks; optionally, after further administering the seventh dose for about 2, about 3, about 4, about 5, about 6, about 7, about 8, about 9, about 10, about 11, about 12, about 13, about 14, about 15, about 16, about 17, about 18, about 19, or about 20 weeks, administering an eighth dose of about 30-84mg of the compound of formula (I), or the pharmaceutically acceptable salt, amide, or ester thereof at a frequency of once weekly or once every two weeks.

In some embodiments, wherein the first dose is about 0.5-5mg, preferably about 1-4.5mg, preferably about 1.5-4mg, preferably about 1.5-3.5mg, preferably about 2-3mg, preferably about 3mg; and/or
the second dose is about 3-7.5mg, preferably about 3.5-7.5mg, preferably about 4-7mg, preferably about 4.5-6.5mg, preferably about 5-6mg, preferably about 6mg; and/or
the third dose is about 5-24mg, preferably about 6-20mg mg, preferably about 6-18mg, preferably about 6-12mg, preferably about 6mg, about 7mg, about 8mg, about 9mg, about 10mg, about 11mg, about 12mg, about 13mg, about 14mg, about 15mg, about 16mg, about 17mg, about 18mg, about 19mg, or about 20mg, preferably about 9mg or about 12mg; and/or
the fourth dose is about 7-40mg, preferably about 9-36mg, preferably about 9mg, about 10mg, about 11mg, about 12mg, about 13mg, about 14mg, about 15mg, about 16mg, about 17mg, about 18mg, about 19mg, about 20mg, about 21mg, about 22mg, about 23mg, about 24mg, about 25mg, about 26mg, about 27mg, about 28mg, about 29mg, about 30mg, about 31mg, about 32mg, about 33mg, about 34mg, about 35mg, or about 36mg, preferably about 12mg or about 24mg; and/or
the fifth dose is about 9-50mg, preferably about 12-48mg, preferably about 12mg, about 13mg, about 14mg, about 15mg, about 16mg, about 17mg, about 18mg, about 19mg, about 20mg, about 21mg, about 22mg, about 23mg, about 24mg, about 25mg, about 26mg, about 27mg, about 28mg, about 29mg, about 30mg, about 31mg, about 32mg, about 33mg, about 34mg, about 35mg, about 36mg, about 37mg, about 38mg, about 39mg, about 40mg, about 41mg, about 42mg, about 43mg, about 44mg, about 45mg, about 46mg, about 47mg or about 48mg, preferably about 15mg, about 18mg or about 36mg; and/or
the sixth dose is about 12-50mg, preferably about 18-60mg, preferably about 24mg, about 25mg, about 26mg, about 27mg, about 28mg, about 29mg, about 30mg, about 31mg, about 32mg, about 33mg, about 34mg, about 35mg, about 36mg, about 37mg, about 38mg, about 39mg, about 40mg, about 41mg, about 42mg, about 43mg, about 44mg, about 45mg, about 46mg, about 47mg, about 48mg, about 49mg, about 50mg, about 51mg, about 52mg, about 53mg, about 54mg, about 55mg, about 56mg, about 57mg, about 58mg, about 59mg, or about 60mg, preferably about 24mg or about 48mg; and/or
the seventh dose is about 15-75mg, preferably about 24-72mg, preferably about 24mg, about 25mg, about 26mg, about 27mg, about 28mg, about 29mg, about 30mg, about 31mg, about 32mg, about 33mg, about 34mg, about 35mg, about 36mg, about 37mg, about 38mg, about 39mg, about 40mg, about 41mg, about 42mg, about 43mg, about 44mg, about 45mg, about 46mg, about 47mg, about 48mg, about 49mg, about 50mg, about 51mg, about 52mg, about 53mg, about 54mg, about 55mg, about 56mg, about 57mg, about 58mg, about 59mg, or about 60mg, preferably about 30mg, about 36mg, or about 48mg; and/or
the eighth dose is about 30-84mg, preferably about 36-72mg, preferably about 30mg, about 31mg, about 32mg, about 33mg, about 34mg, about 35mg, about 36mg, about 37mg, about 38mg, about 39mg, about 40mg, about 41mg, about 42mg, about 43mg, about 44mg, about 45mg, about 46mg, about 47mg, about 48mg, about 49mg, about 50mg, about 51mg, about 52mg, about 53mg, about 54mg, about 55mg, about 56mg, about 57mg, about 58mg, about 59mg, about 60mg, about 61mg, about 62mg, about 63mg, about 64mg, about 65mg, about 66mg, about 67mg, about 68mg, about 69mg, about 70mg, about 71mg or about 72mg, preferably about 48mg.

In some embodiments, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 weeks or about 4 weeks, administering a second dose of about 6mg of the compound of formula (I), or the pharmaceutically acceptable salt, amide or ester thereof at a frequency of once weekly or once every two weeks;
after administering the second dose for about 4 weeks, administering a third dose of about 9mg of the compound of formula (I), or the pharmaceutically acceptable salt, amide, or ester thereof at a frequency of once weekly or once every two weeks; and
after administering the third dose for about 4 weeks, administering a fourth dose of about 12mg of the compound of formula (I), or the pharmaceutically acceptable salt, amide or ester thereof at a frequency of once weekly or once every two weeks, preferably administering the fourth dose of about 12mg of the compound of formula (I), or the pharmaceutically acceptable salt, amide or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably, administering the fourth dose of about 12mg of the compound of formula (I), or the pharmaceutically acceptable salt, amide, or ester thereof at the frequency of once weekly or once every two weeks for about 20 weeks.

In some embodiments, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 or 4 weeks, administering a second dose of about 6mg of the compound of formula (I), or the pharmaceutically acceptable salt, amide or ester thereof at a frequency of once weekly or once every two weeks;
after administering the second dose for about 4 weeks, administering a third dose of about 9mg of the compound of formula (I), or the pharmaceutically acceptable salt, amide, or ester thereof at a frequency of once weekly or once every two weeks;
after administering the third dose for about 4 weeks, administering a fourth dose of about 12mg of the compound of formula (I), or the pharmaceutically acceptable salt, amide, or ester thereof at a frequency of once weekly or once every two weeks; and
after administering the fourth dose for about 4 weeks, administering a fifth dose of about 18mg of the compound of formula (I), or the pharmaceutically acceptable salt, amide, or ester thereof at a frequency of once weekly or once every two weeks, preferably, administering the fifth dose of about 18mg of the compound of formula (I), or the pharmaceutically acceptable salt, amide, or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably, administering the fifth dose of about 18mg of the compound of formula (I), or the pharmaceutically acceptable salt, amide, or ester thereof at the frequency of once weekly or once every two weeks for about 16 weeks.

In some embodiments, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 or 4 weeks, administering a second dose of about 6mg of the compound of formula (I), or the pharmaceutically acceptable salt, amide, or ester thereof at a frequency of once weekly or once every two weeks;
after administering the second dose for about 4 weeks, administering a third dose of about 9mg of the compound of formula (I), or the pharmaceutically acceptable salt, amide, or ester thereof at a frequency of once weekly or once every two weeks;
after administering the third dose for about 4 weeks, administering a fourth dose of about 12mg of the compound of formula (I), or the pharmaceutically acceptable salt, amide, or ester thereof at a frequency of once weekly or once every two weeks;
after administering the fourth dose for about 4 weeks, administering a fifth dose of about 18mg of the compound of formula (I), or the pharmaceutically acceptable salt, amide, or ester thereof at a frequency of once weekly or once every two weeks; and
after administering the fifth dose for about 4 weeks, administering a sixth dose of about 24mg of the compound of formula (I), or the pharmaceutically acceptable salt, amide, or ester thereof at a frequency of once weekly or once every two weeks, preferably, administering the sixth dose of about 24mg of the compound of formula (I), or the pharmaceutically acceptable salt, amide, or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably, administering the sixth dose of about 24mg of the compound of formula (I), or the pharmaceutically acceptable salt, amide, or ester thereof at the frequency of once weekly or once every two weeks for about 12 weeks.

In some embodiments, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 or 4 weeks, administering a second dose of about 6mg of the compound of formula (I), or the pharmaceutically acceptable salt, amide, or ester thereof at a frequency of once weekly or once every two weeks;
after administering the second dose for about 4 weeks, administering a third dose of about 9mg of the compound of formula (I), or the pharmaceutically acceptable salt, amide, or ester thereof at a frequency of once weekly or once every two weeks;
after administering the third dose for about 4 weeks, administering a fourth dose of about 12mg of the compound of formula (I), or the pharmaceutically acceptable salt, amide, or ester thereof at a frequency of once weekly or once every two weeks;
after administering the fourth dose for about 4 weeks, administering a fifth dose of about 18mg of the compound of formula (I), or the pharmaceutically acceptable salt, amide, or ester thereof once weekly or once every two weeks;
after administering the fifth dose for about 4 weeks, administering a sixth dose of about 24mg of the compound of formula (I), or the pharmaceutically acceptable salt, amide, or ester thereof once weekly or once every two weeks; and
after administering the sixth dose for about 4 weeks, administering a seventh dose of about 48mg of the compound of formula (I), or the pharmaceutically acceptable salt, amide or ester thereof at a frequency of once weekly or once every two weeks, preferably administering the seventh dose of about 48mg of the compound of formula (I), or the pharmaceutically acceptable salt, amide or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more;
preferably:
   the method comprises administering to a subject in need thereof the first dose of about 3mg of the compound of formula (I), or the pharmaceutically acceptable salt, amide or ester thereof at the frequency of once weekly or once every two weeks;
   after administering the first dose for about 2 or 4 weeks, administering the second dose of about 6mg of the compound of formula (I), or the pharmaceutically acceptable salt, amide, or ester thereof at the frequency of once weekly or once every two weeks;
   after administering the second dose for about 4 weeks, administering the third dose of about 9mg of the compound of formula (I), or the pharmaceutically acceptable salt, amide, or ester thereof at the frequency of once weekly or once every two weeks;
   after administering the third dose for about 4 weeks, administering the fourth dose of about 12mg of the compound of formula (I), or the pharmaceutically acceptable salt, amide, or ester thereof at the frequency of once weekly or once every two weeks;
   after administering the fourth dose for about 4 weeks, administering the fifth dose of about 18mg of the compound of formula (I), or the pharmaceutically acceptable salt, amide, or ester thereof at the frequency of once weekly or once every two weeks;
   after administering the fifth dose for about 4 weeks, administering the sixth dose of about 24mg of the compound of formula (I), or the pharmaceutically acceptable salt, amide, or ester thereof at the frequency of once weekly or once every two weeks; and
   after administering the sixth dose for about 4 weeks, administering the seventh dose of about 36mg of the compound of formula (I), or the pharmaceutically acceptable salt, amide, or ester thereof at the frequency of once weekly or once every two weeks;
   after administering the seventh dose for about 4 weeks, administering the eighth dose of about 48mg of the compound of formula (I), or the pharmaceutically acceptable salt, amide or ester thereof at the frequency of once weekly or once every two weeks, preferably administering the eighth dose of about 48mg of the compound of formula (I), or the pharmaceutically acceptable salt, amide or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more.

In some embodiments, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 or 4 weeks, administering a second dose of about 6mg of the compound of formula (I), or the pharmaceutically acceptable salt, amide, or ester thereof at a frequency of once weekly or once every two weeks;
after administering the second dose for about 4 weeks, administering a third dose of about 12mg of the compound of formula (I), or the pharmaceutically acceptable salt, amide or ester thereof at a frequency of once weekly or once every two weeks;
after administering the third dose for about 4 weeks, administering a fourth dose of about 24mg of the compound of formula (I), or the pharmaceutically acceptable salt, amide or ester thereof at a frequency of once weekly or once every two weeks;
after administering the fourth dose for about 4 weeks, administering a fifth dose of about 36mg of the compound of formula (I), or the pharmaceutically acceptable salt, amide or ester thereof at a frequency of once weekly or once every two weeks; and
after administering the fifth dose for about 4 weeks, administering a sixth dose of about 48mg of the compound of formula (I) or the pharmaceutically acceptable salt, amide or ester thereof at a frequency of once weekly or once every two weeks, preferably administering the sixth dose of about 48mg of the compound of formula (I), or the pharmaceutically acceptable salt, amide or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably administering the sixth dose of about 48mg of the compound of formula (I), or the pharmaceutically acceptable salt, amide or ester thereof at the frequency of once weekly or once every two weeks for 12 weeks or more.

In some embodiments, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 or 4 weeks, administering a second dose of about 6mg of the compound of formula (I), or the pharmaceutically acceptable salt, amide, or ester thereof at a frequency of once weekly or once every two weeks;
after administering the second dose for about 4 weeks, administering a third dose of about 9mg of the compound of formula (I), or the pharmaceutically acceptable salt, amide, or ester thereof at a frequency of once weekly or once every two weeks;
after administering the third dose for about 4 weeks, administering a fourth dose of about 12mg of the compound of formula (I), or the pharmaceutically acceptable salt, amide, or ester thereof at a frequency of once weekly or once every two weeks;
after administering the fourth dose for 4 weeks, administering a fifth dose of about 15mg of the compound of formula (I), or the pharmaceutically acceptable salt, amide or ester thereof at a frequency of once weekly or once every two weeks; and
after administering the fifth dose for about 4 weeks, administering a sixth dose of about 24mg of the compound of formula (I), or the pharmaceutically acceptable salt, amide, or ester thereof at a frequency of once weekly or once every two weeks, preferably administering the six dose of about 24mg of the compound of formula (I), or the pharmaceutically acceptable salt, amide, or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably, administering the sixth dose of about 24mg of the compound of formula (I), or the pharmaceutically acceptable salt, amide or ester thereof at the frequency of once weekly or once every two weeks for about 12 weeks or more.

In some embodiments, wherein the method comprises administering to a subject in need thereof a first' dose of about 0.25-3mg of the compound of formula (I), or the pharmaceutically acceptable salt, amide or ester thereof at a frequency of once weekly or once every two weeks; and/or
after administering the first' dose for about 1, about 2, about 3, or about 4 weeks, administering a second' dose of about 1-5mg of the compound of formula (I), or the pharmaceutically acceptable salt, amide, or ester thereof at a frequency of once weekly or once every two weeks; and/or
after administering the second' dose for about 2, about 3, about 4, about 5, about 6, about 7, or about 8 weeks, administering a third' dose of about 3-7mg of the compound of formula (I), or the pharmaceutically acceptable salt, amide, or ester thereof at a frequency of once weekly or once every two weeks; and/or
after administering the third' dose for about 2, about 3, about 4, about 5, about 6, about 7, or about 8 weeks, administering a fourth' dose of about 5-9mg of the compound of formula (I), or the pharmaceutically acceptable salt, amide, or ester thereof at a frequency of once weekly or once every two weeks; and/or
after administering the fourth' dose for about 2, about 3, about 4, about 5, about 6, about 7, about 8, about 9, about 10, about 11, about 12, about 13, about 14, about 15, about 16, about 17, about 18, about 19, or about 20 weeks, administering a fifth' dose of about 7-12mg of the compound of formula (I), or the pharmaceutically acceptable salt, amide, or ester thereof at a frequency of once weekly or once every two weeks; and/or after administering the fifth' dose for about 2, about 3, about 4, about 5, about 6, about 7, about 8, about 9, about 10, about 11, about 12, about 13, about 14, about 15, about 16, about 17, about 18, about 19, or about 20 weeks, administering a sixth' dose of about 9-15mg of the compound of formula (I), or the pharmaceutically acceptable salt, amide, or ester thereof at a frequency of once weekly or once every two weeks; and/or
after administering the sixth' dose for about 2, about 3, about 4, about 5, about 6, about 7, about 8, about 9, about 10, about 11, about 12, about 13, about 14, about 15, about 16, about 17, about 18, about 19, or about 20 weeks, administering a seventh' dose of about 12-18mg of the compound of formula (I), or the pharmaceutically acceptable salt, amide, or ester thereof at a frequency of once weekly or once every two weeks; and/or
after administering the seventh' dose for about 2, about 3, about 4, about 5, about 6, about 7, about 8, about 9, about 10, about 11, about 12, about 13, about 14, about 15, about 16, about 17, about 18, about 19, or about 20 weeks, administering an eighth' dose of about 15-24mg of the compound of formula (I), or the pharmaceutically acceptable salt, amide, or ester thereof at a frequency of once weekly or once every two weeks; and/or
after administering the eighth' dose for about 2, about 3, about 4, about 5, about 6, about 7, about 8, about 9, about 10, about 11, about 12, about 13, about 14, about 15, about 16, about 17, about 18, about 19, or about 20 weeks, administering a ninth' dose of about 18-30mg of the compound of formula (I), or the pharmaceutically acceptable salt, amide, or ester thereof at a frequency of once weekly or once every two weeks; and/or
after administering the ninth' dose for about 2, about 3, about 4, about 5, about 6, about 7, about 8, about 9, about 10, about 11, about 12, about 13, about 14, about 15, about 16, about 17, about 18, about 19, or about 20 weeks, administering a tenth' dose of about 24-48mg of the compound of formula (I), or the pharmaceutically acceptable salt, amide, or ester thereof at a frequency of once weekly or once every two weeks.

In some embodiments, wherein the first' dose is about 0.5-3mg, preferably about 0.5mg, about 1mg, about 1.5mg, about 2mg, about 2.5mg, or about 3mg, preferably about 1.5mg; and/or
the second' dose is about 1-5mg, preferably about 1mg, about 2mg, about 3mg, about 4mg, or about 5mg, preferably about 3mg; and/or
the third' dose is about 3-7mg, preferably about 3mg, about 4mg, about 5mg, about 6mg, or about 7mg, preferably about 5mg; and/or
the fourth' dose is about 5-9mg, preferably about 5mg, about 6mg, about 7mg, about 8mg, about 9mg, preferably about 6mg or about 7mg; and/or
the fifth' dose is about 7-12mg, preferably about 7mg, about 8mg, about 9mg, about 10mg, about 11mg, or about 12mg, preferably about 9mg; and/or
the sixth' dose is about 9-15mg, preferably about 9mg, about 10mg, about 11mg, about 12mg, about 13mg, about 14mg, or about 15mg, preferably about 12mg; and/or
the seventh' dose is about 12-18mg, preferably about 12mg, about 13mg, about 14mg, about 15mg, about 16mg, about 17mg, or about 18mg, preferably about 15mg; and/or
the eighth' dose is about 15-24mg, preferably about 15mg, about 16mg, about 17mg, about 18mg, about 19mg, about 20mg, about 21mg, about 22mg, about 23mg, or about 24mg, preferably about 18mg; and/or
the ninth' dose is about 18-30mg, preferably about 18mg, about 19mg, about 20mg, about 21mg, about 22mg, about 23mg, about 24mg, about 25mg, about 26mg, about 27mg, about 28mg, about 29mg, about 30mg, preferably about 24mg; and/or
the tenth' dose is about 24-48mg, preferably about 24mg, about 25mg, about 26mg, about 27mg, about 28mg, about 29mg, about 30mg, about 31mg, about 32mg, about 33mg, about 34mg, about 35mg, about 36mg, about 37mg, about 38mg, about 39mg, about 40mg, about 41mg, about 42mg, about 43mg, about 44mg, about 45mg, about 46mg, about 47mg, or about 48mg, preferably about 30mg.

In some embodiments, wherein the method comprises administering to a subject in need thereof a first' dose of about 1.5mg of the compound of formula (I), or the pharmaceutically acceptable salt, amide or ester thereof at a frequency of once weekly or once every two weeks;
after administering the first' dose for about 1 week, administering a second' dose of about 3mg of the compound of formula (I), or the pharmaceutically acceptable salt, amide or ester thereof at a frequency of once weekly;
after administering the second' dose for about 2 weeks, administering a third' dose of about 5mg of the compound of formula (I), or the pharmaceutically acceptable salt, amide or ester thereof at a frequency of once weekly;
after administering the third' dose for about 4 weeks, administering a fourth' dose of about 7mg of the compound of formula (I), or the pharmaceutically acceptable salt, amide or ester thereof at a frequency of once weekly;
after administering the fourth' dose for about 4 weeks, administering a fifth' dose of about 9mg of the compound of formula (I), or the pharmaceutically acceptable salt, amide or ester thereof at a frequency of once weekly or once every two weeks;
after administering the fifth' dose for about 4 weeks, administering a sixth' dose of about 12mg of the compound of formula (I), or the pharmaceutically acceptable salt, amide or ester thereof at a frequency of once weekly or once every two weeks;
after administering the sixth' dose for about 4 weeks, administering a seventh' dose of about 15mg of the compound of formula (I), or the pharmaceutically acceptable salt, amide or ester thereof at a frequency of once weekly or once every two weeks;
after administering the seventh' dose for about 4 weeks, administering a eighth' dose of about 18mg of the compound of formula (I), or the pharmaceutically acceptable salt, amide or ester thereof at a frequency of once weekly or once every two weeks;
after administering the eighth' dose for about 4 weeks, administering a ninth' dose of about 24mg of the compound of formula (I), or the pharmaceutically acceptable salt, amide or ester thereof at a frequency of once weekly or once every two weeks; and
after administering the ninth' dose for about 4 weeks, administering a tenth' dose of about 30mg of the compound of formula (I), or the pharmaceutically acceptable salt, amide or ester thereof at a frequency of once weekly or once every two weeks, preferably administering the tenth' dose of about 30mg of the compound of formula (I), or the pharmaceutically acceptable salt, amide or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably administering the tenth' dose of about 30mg of the compound of formula (I), or the pharmaceutically acceptable salt, amide or ester thereof at a frequency of once weekly or once every two weeks for about 4 weeks.

In some embodiments, the compound of formula (I), or the pharmaceutically acceptable salt, amide or ester thereof, is administered parenterally, preferably subcutaneously.

In some embodiments, the compound of formula (I), or the pharmaceutically acceptable salt, amide or ester thereof is administered for 10 weeks or more, preferably 15 weeks or more, preferably 20 weeks or more, preferably 25 weeks or more, preferably 30 weeks or more, preferably 35 weeks or more, preferably 12 months or more, preferably 16 months or more, preferably 18 months or more, preferably 24 months or more, preferably 28 months or more, preferably 29 months or more, preferably 30 months or more.

In some embodiments, in the method mentioned above, characterized in that, the subject has a BMI ≥ 24kg/m², preferably BMI ≥ 25kg/m², preferably BMI ≥ 26kg/m²;
preferably:
the subject is accompanied by at least one of the following manifestations:
1) comorbidity with one or more of prediabetes, hypertension, dyslipidemia, or fatty liver;
2) comorbidity with weight-bearing joint pain; and
3) obesity-induced dyspnoea or obstructive sleep apnea syndrome.

In some embodiments, in the method mentioned above, the compound of formula (I), or the pharmaceutically acceptable salt, amide or ester thereof, is administered in the form of a pharmaceutical composition comprising one or more pharmaceutically acceptable excipients.

In some embodiments, the pharmaceutical composition comprises:
about 3mg/ml, about 6mg/ml, about 12mg/ml, about 18mg/ml, about 24mg/ml, about 36mg/ml, or about 48mg/ml of the compound of formula (I), or the pharmaceutically acceptable salt, amide, or ester thereof;
about 5mg/ml, about 6mg/ml, about 7mg/ml, about 8mg/ml, about 9mg/ml or about 10mg/ml of NaCl, preferably about 7mg/ml of NaCl;
about 1mM, 2mM, 3mM, 4mM, 5mM, 6mM, 7mM, 8mM, 9mM or 10mM of citric acid;
about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of a Na₂HPO₄ solution, preferably about 1.42 mg/ml of Na₂HPO₄; and
the pH of the pharmaceutical composition is about 7.2, about 7.3, about 7.4, about 7.5, about 7.6, about 7.7, about 7.8, about 7.9, about 8.0, about 8.1, or about 8.2, preferably about 7.3.

In some embodiments, after administration of the compound of formula (I) or the pharmaceutically acceptable salt, amide, or ester thereof, a subject achieves a weight loss of about 5% or more, preferably about 10%-60%, preferably about 10%-50%, preferably about 10%-40%, preferably about 10%-30%, preferably about 10%-25%, preferably about 10%, about 10.16%, about 11%, about 11.15%, about 12%, about 12.23%, about 13%, about 13.22%, about 13.26%, about 13.5%, about 14%, about 14.25%, about 15%, about 16%, about 16.3%, about 16.79%, about 17%, about 17.29%, about 17.78%, about 18%, about 18.6%, about 19%, or about 20%; preferably, after administering the compound of formula (I) or the pharmaceutically acceptable salt, amide, or ester thereof for about 10 weeks, about 20 weeks, about 25 weeks, about 30 weeks or about 35 weeks, the subject achieves a weight loss of about 10% or more, preferably about 10%-30%, preferably about 10%-25%, preferably about 10%, about 10.16%, about 11%, about 11.15%, about 12%, about 12.23%, about 13%, about 13.22%, about 13.26%, about 13.5%, about 14%, about 14.25%, about 15%, about 16%, about 16.3%, about 16.79%, about 17%, about 17.29%, about 17.78%, about 18%, about 18.6%, about 19%, or about 20%.

In some embodiments, after administration of the compound of formula (I) or the pharmaceutically acceptable salt, amide, or ester thereof, a subject achieves a weight loss of about 10% or more, preferably about 10%-30%, preferably about 10%-25%, preferably about 10%, about 11%, about 12%, about 13%, about 13.5%, about 14%, about 15%, about 16%, about 17%, about 18%, about 18.6%, about 19%, or about 20%; preferably, after administering the compound of formula (I) or the pharmaceutically acceptable salt, amide, or ester thereof for about 10 weeks, about 20 weeks, about 25 weeks, about 30 weeks or about 35 weeks, a subject achieves a weight loss of about 10% or more, preferably about 10%-30%, preferably about 10%-25%, preferably about 10%, about 11%, about 12%, about 13%, about 13.5%, about 14%, about 15%, about 16%, about 17%, about 18%, about 18.6%, about 19%, or about 20%.

In some embodiments, when the fourth dose of about 12mg of the compound of formula (I), or the pharmaceutically acceptable salt, amide or ester thereof, is administered at the frequency of every two weeks for 20 weeks or more, the average body weight of the subject is reduced by about 11.15% or more compared to baseline.

In some embodiments, when the fifth dose of about 18mg of the compound of formula (I), or the pharmaceutically acceptable salt, amide or ester thereof, is administered at the frequency of every two weeks for 16 weeks or more, the average body weight of the subject is reduced by about 13.22% or more compared to baseline.

In some embodiments, when the sixth dose of about 24mg of the compound of formula (I), or the pharmaceutically acceptable salt, amide or ester thereof, is administered at the frequency of every two weeks for 12 weeks or more, the average body weight of the subject is reduced by about 14.25% or more compared to baseline.

In some embodiments, when the seventh dose of about 48mg of the compound of formula (I), or the pharmaceutically acceptable salt, amide or ester thereof, is administered at the frequency of every two weeks for 8 weeks or more, the average body weight of the subject is reduced by about 17.29% or more compared to baseline.

In some embodiments, when the sixth dose of about 24mg of the compound of formula (I), or the pharmaceutically acceptable salt, amide or ester thereof, is administered at the frequency of once weekly for 12 weeks or more, the average body weight of the subject is reduced by about 17.78% or more compared to baseline.

### Definitions

To better understand the present invention, definitions and explanations of relevant terms are provided below.

### GLP-1 analogs, and GLP-1 derivatives

The terms "GLP-1 analog" or "analog of GLP-1" as used herein refer to a peptide or compound which is a variant of human glucagon-like peptide-1 (GLP-1(7-37)), wherein one or more amino acid residues of GLP-1(7-37) have been substituted, and/or one or more amino acid residues have been deleted, and/or one or more amino acid residues have been added. Specifically, the sequence of GLP-1(7-37) is shown in SEQ ID NO: 1 in the sequence listing. A peptide having the sequence shown in SEQ ID NO: 1 may also be referred to as "native" GLP-1 or "native" GLP-1(7-37).

In the sequence listing, the first amino acid residue (histidine) of SEQ ID NO:1 is numbered 1. However, in the following text, following established convention in the art, this histidine residue is numbered 7, and the subsequent amino acid residues are numbered accordingly, concluding with glycine as number 37. Therefore, typically, the amino acid residue numbering or position numbering for the GLP-1(7-37) sequence discussed herein refers to the sequence starting with His at position 7 and ending with Gly at position 37.

[Gly8, Arg34] GLP-1-(7-37) peptide is a GLP-1 analog wherein Gly and Arg are substituted at positions 8 and 34, respectively, relative to GLP-1(7-37) (SEQ ID NO: 1), and [Arg34] GLP-1-(7-37) peptide is a GLP-1 analog having Arg at position 34 corresponding to GLP-1(7-37) (SEQ ID NO: 1). Specifically, the amino acid sequence of the [Gly8, Arg34] GLP-1-(7-37) peptide is shown as SEQ ID NO: 2 in the sequence listing.

In the context of GLP-1 peptides or their analogs, the term "derivative" as used herein refers to a chemically modified GLP-1 peptide or analog wherein one or more substituents are covalently bonded to said peptides. Such substituents may also be referred to as side chains.

Unless otherwise specified, references to acylation of lysine residues should be understood as occurring at the ε-amino group.

The term "peptide", when used in reference to GLP-1 analogs such as those of the present invention, refers to a compound comprising a sequence of amino acids interconnected by amide (or peptide) bonds.

In one specific embodiment, the peptide is largely or primarily composed of amino acids interconnected via amide bonds (e.g., at least 50%, 60%, 70%, 80%, or at least 90% by molar mass). In another specific embodiment, the peptide is composed of amino acids interconnected via peptide bonds.

Amino acids are molecules comprising both an amino group and a carboxyl group, optionally comprising one or more additional groups, commonly referred to as side chains.

The term "amino acid" comprises proteinogenic amino acids (encoded by the genetic code, including natural amino acids and standard amino acids), non-proteinogenic amino acids (not found in proteins, and/or not encoded in the standard genetic code), and synthetic amino acids. Non-proteinogenic amino acids are those that can be incorporated into peptides via peptide bonds but are not proteinogenic amino acids. Synthetic non-proteinogenic amino acids include those produced through chemical synthesis, namely D-isomers of amino acids encoded by the genetic code such as D-alanine and D-leucine, Aib (α-aminoisobutyric acid), Abu (α-aminobutyric acid), 3-aminomethylbenzoic acid, o-aminobenzoic acid, deamidated histidine, β-analogues of amino acids such as β-alanine, D-histidine, deamidated histidine, 2-aminohistidine, β-hydroxyhistidine, and homohistidine, etc.

Non- limiting examples of amino acids not encoded by the genetic code include γ-carboxyglutamic acid, ornithine, D-alanine, D-glutamine, and phosphoserine. Non-limiting examples of synthetic amino acids include D-isomers of amino acids, such as D-alanine and D-leucine, Aib (α-aminoisobutyric acid), β-alanine, and des-amino-histidine (desH, also known as imidopropionic acid, abbreviated as Imp).

In the following text, all amino acids not explicitly labeled as optical isomers are understood to refer to the L-isomer (unless otherwise specified).

In this application, when referring to specifically enumerated values or value ranges, the term "about" as used herein generally means within 20% of the given value or range, preferably within 10%, and more preferably within 5%.

The term "treatment" comprises therapeutic treatments, preventive treatments, and uses in reducing subjects' risk of developing diseases or other risk factors. Treatment includes, but is not limited to, the complete cure of diseases, as well as the alleviation of symptoms or mitigation of underlying risks.

### Detailed Description

The following detailed description of the embodiment of the present invention will be provided with reference to specific examples. However, those skilled in the art will understand that the following examples are provided merely to illustrate the invention and should not be construed as limiting the scope of the invention. Unless otherwise specified in the examples, standard conditions or manufacturer-recommended conditions shall be followed. Unless otherwise indicated, the reagents or instruments used for which the manufacturer is not specified are all conventional commercially available products.

Abbreviations
AE: Adverse event
AESI: Adverse event of special interest
AUC: Area Under the drug concentration-time curveHbA1c: Glycosylated Hemoglobin
GLP-1: Glucagon-like peptide-1
BMI: Body mass index
N: Number of subjects

### Example 1

### Preparation of the title compound: N-ε²⁶-[2-(2-[2-(2-[2-(2-[4-(21-carboxyheneicosanoylamino)-4(S)-carboxybutanoylamino]ethoxy)ethoxy]acetyl amino)ethoxy]ethoxy)acetyl][Gly8, Arg34]GLP-1-(7-37) peptide (Compound (I))

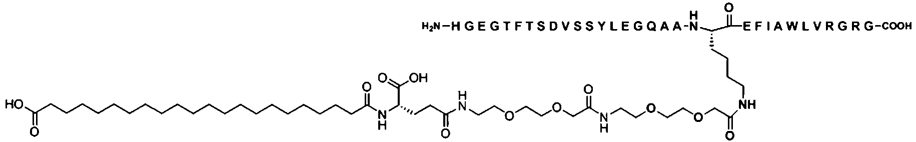

Compound I is a GLP-1 receptor agonist with CAS Registry Number: 2668298-70-0, and can be prepared with reference to, for example, the methods described in WO2021136303A1.

### Example 2: Pharmaceutical Compositions of Compound (I)

Preparation of pharmaceutical composition 1 of compound (I): Compound (I) at various concentrations (15 mg/3 ml or 90 mg/3 ml), 14 mg/ml propylene glycol, 1.42 mg/ml anhydrous disodium hydrogen phosphate; and pH 7.3.

Preparation of pharmaceutical composition 2 of compound (I): Compound (I) at various concentrations (15 mg/3 ml or 90 mg/3 ml), 14 mg/ml propylene glycol, 1.42 mg/ml anhydrous disodium hydrogen phosphate, approximately 5 mM citric acid; and pH 7.3.

Preparation of pharmaceutical composition 3 of compound (I): Compound (I) at various concentrations (15 mg/3 ml or 90 mg/3 ml), 8.25 mg/ml (141.17 mM) sodium chloride, 1.42 mg/ml anhydrous disodium hydrogen phosphate, approximately 5 mM citric acid; and pH 7.3.

### Example 3: Weight Loss Efficacy of Compound (I) Injection

A Multicenter, Randomized, Placebo-Controlled Phase II Clinical Study to Evaluate the Efficacy and Safety of Compound (I) Injection in Chinese Adult Obesity/overweight Subjects. The primary objective of the Phase II clinical trial is to evaluate the weight loss effect of Compound (I) Injection in adult obesity/overweight subjects.

Investigational Drug, Dosage, Administration Method, and Batch Number:
Drug Name: Compound (I) Injection
Specification: 3 mL/vial (5 mg/mL or 30 mg/mL)
Batch Numbers: P3AI122001 / P3AI423001
Expiration Date: October 2024 / April 2025
Administration Method: Subcutaneous injection (allow to equilibrate to room temperature before injection)
Manufactured and supplied by Gan & Lee Pharmaceuticals Co., Ltd.
Control Drug, Dosage, Administration Method, and Batch Number:
   Drug Name: Placebo
   Specification: 3 mL/vial (does not contain Compound (I))
   Batch Numbers: P3AIM21003 / P3AI4M23001
   Expiration Date: August 2026 / April 2028
   Administration Method: Subcutaneous injection (allow to equilibrate to room temperature before injection)

### Manufactured and supplied by Gan & Lee Pharmaceuticals Co., Ltd.

Study Methods:
This study is a multicenter, randomized, placebo-controlled Phase II clinical trial conducted in Chinese adult obesity/overweight subjects, aimed at evaluating the efficacy of Compound (I) Injection in adult obesity/overweight subjects. The trial enrolled adult obesity/overweight subjects with inadequate response to diet and exercise management.

Eligible subjects who passed screening were assigned to 5 different treatment groups: 4 groups receiving subcutaneous injections of Compound (I) Injection (or corresponding volume of placebo) once every 2 weeks at different target or maintenance doses (12 mg, 18 mg, 24 mg, 48 mg), and one group receiving subcutaneous injections of Compound (I) Injection (or corresponding volume of placebo) once weekly at a target dose (24 mg*). All subjects received standard diet and exercise guidance after randomization.

Each subject participated in the study for up to 34 weeks, including a 1-week screening period, a 30-week treatment period/study visits (including a 10- to 18- week dose adjustment period and a 12- to 20- week stable dose maintenance period), and a 3-week safety follow-up period.

The placebo is the vehicle excipient for Compound (I), which does not contain Compound (I) but has other components similar to those in Compound (I) Injection. The appearance, weight, odor, and other characteristics of the placebo are similar to those of the investigational drug. It is manufactured and supplied by Gan & Lee Pharmaceuticals Co., Ltd.

As shown in Table 1, subjects were randomized to receive Compound (I) Injection or an equivalent volume of placebo according to the dosing regimens specified in Table 1.

**Table 1: Dosing Regimens**

| Group | Dosing Freque ncy | Target Dose | Dosing Design | | | Number of Subjects | |
|---|---|---|---|---|---|---|---|
| | | | Duration (weeks) | Dosing Weeks | Dose | Compound (I) Injection | Placebo |
| 1 | Every two weeks | Compou nd (I)-12 mg | 2 | WO | 3 mg | 52 | 66 |
| | | | 4 | W2/4 | 6 mg | | |
| | | | 4 | W6/8 | 9 mg | | |
| | | | 20 | W10/12/14/16/18/20/22/24/26/2 8 | 12 mg | | |
| 2 | | Compou nd (I)-18 mg | 2 | WO | 3 mg | 53 | |
| | | | 4 | W2/4 | 6 mg | | |
| | | | 4 | W6/8 | 9 mg | | |
| | | | 4 | W10/12 | 12 mg | | |
| | | | 16 | W14/16/18/20/22/24/26/28 | 18 mg | | |
| 3 | | Compound (I)-24 mg | 2 | WO | 3 mg | 52 | |
| | | | 4 | W2/4 | 6 mg | | |
| | | | 4 | W6/8 | 9 mg | | |
| | | | 4 | W10/12 | 12 mg | | |
| | | | 4 | W14/16 | 18 mg | | |
| | | | 12 | W18/20/22/24/26/28 | 24 mg | | |
| 4 | | Compoun d (I)-48 mg | 2 | WO | 3 mg | 64 | |
| | | | 4 | W2/4 | 6 mg | | |
| | | | 4 | W6/8 | 12 mg | | |
| | | | 4 | W10/12 | 24mg | | |
| | | | 4 | W14/16 | 36 mg | | |
| | | | 12 | W18/20/22/24/26/28 | 48 mg | | |
| 5 | Once weekly | Compo und (I)-24 mg(or 18mg) * | 2 | WO/1 | 3 mg | 53 | |
| | | | 4 | W2/3/4/5 | 6 mg | | |
| | | | 4 | W6/7/8/9 | 9 mg | | |
| | | | 4 | W10/11/12/13 | 12 mg | | |
| | | | 4 | W14/15/16/17 | 15 mg | | |
| | | | 12 | W18/19/20/21/22/23/24/25/26/2 7/28/29 | 24 mg | | |

| | | | | | | | |
|---|---|---|---|---|---|---|---|
| **Note:** W stands for week; W2/4 indicates Week 2 or Week 4. | | | | | | | |

Compound (I) - 24 mg (or 18 mg) 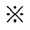 indicates that subjects from the previous once-weekly 18 mg dosing group who do not consent to enter the once-weekly 24 mg dosing group will continue to receive the 18 mg dose from W18 to W29.

**Table 2. Subject Inclusion and Exclusion Criteria (Eligible subjects must meet all inclusion criteria; subjects meeting one or more exclusion criteria will be excluded)**

| | Limit |
|---|---|
| **Inclusion Criteria** | Subjects must meet all of the following inclusion criteria to be enrolled in the study: |
| | 1. Aged 18 to 75 years (inclusive), male or female; |
| | 2. Participants who were obese (BMI ≥ 28 kg/m²), or overweight (24 kg/m² ≤ BMI < 28 kg/m²) with at least one of the following manifestations: |
| | 1) comorbidity with one or more of prediabetes, hypertension, dyslipidemia, or fatty liver; |
| | 2) comorbidity with weight-bearing joint pain; |
| | 3) Obesity-induced dyspnoea or obstructive sleep apnea syndrome. |
| | 3. Able to understand the procedures and methods of this study, willing and able to maintain a stable diet and exercise lifestyle throughout the study period, and voluntarily sign the informed consent form. |
| Exclusion Criteria | Subjects meeting any of the following criteria will not be enrolled in the study: |
| | 1. Presence of limb deformities or defects that affect the measurement of height and weight. |
| | 2. Women who are pregnant or breastfeeding; men or women of reproductive potential who are unwilling to use contraception throughout the entire study period and for 6 months after the last dose of the investigational product. |
| | 3. History of drug abuse within 1 year prior to screening, or a positive result on the drug abuse screening during screening or before randomization. |
| | 4. History of alcohol abuse within 6 months prior to screening, defined as an average weekly |
| | alcohol intake exceeding 14 units (males) / 7 units (females) (1 standard unit is equivalent to 360 mL of beer, or 150 mL of wine, or 45 mL of 40% distilled spirits). |
| | 5. Known or suspected allergy to GLP-1 receptor agonists or to any of the excipients. |
| | 6. Subjects with an absolute change in body weight > 5.0% for any reason within 3 months prior to screening. Body weight change is calculated as: (highest weight - lowestweight within the 3 months prior to screening) / highest weight * 100%. |
| | 7. History or evidence of any of the following diseases: |
| | 1) Previously diagnosed with Type 1 or Type 2 diabetes mellitus; |
| | 2) Subjects with proliferative retinopathy within 1 year prior to screening or at screening; |
| | 3) History of severe hypoglycemia or recurrent (≥ 2 times within six months) symptomatic hypoglycemia; |
| | 4) Obesity secondary to other diseases or medications, including: elevated cortisol hormones (e.g., Cushing's syndrome), obesity due to pituitary or hypothalamic injury, obesity resulting from dose reduction or discontinuation of weight-loss drugs, etc.; |
| | 5) History of moderate to severe depressive disorder, or other severe psychiatric disorders (schizophrenia, bipolar disorder, etc.), or a Patient Health Questionnaire (PHQ-9) score ≥ 15 at screening; |
| | 6) History of suicidal ideation or suicidal behavior, or presence of suicidal ideation of Type 4 or 5 on the Columbia-Suicide Severity Rating Scale (C-SSRS) at screening; |
| | 7) Systolic blood pressure ≥ 160 mmHg and/or diastolic blood pressure ≥ 100 mmHg at screening; |
| | 8) History of medullary thyroid carcinoma (MTC), multiple endocrine neoplasia (MEN) 2A or 2B, or relevant family history, or history of malignant tumors within the previous 5 years (except for cured basal cell carcinoma of the skin, squamous cell carcinoma of the skin, other polyps, or carcinoma in situ of the cervix); |
| | 9) History of the following cardiovascular or cerebrovascular diseases: decompensated heart failure (New York Heart Association [NYHA] Class III or IV), unstable angina pectoris or myocardial infarction, history of heart valve replacement surgery, history of coronary artery bypass grafting (CABG) or other invasive cardiovascular procedures including percutaneous coronary intervention, cerebrovascular accident or stroke with sequelae; |
| | 10) History of acute or chronic pancreatitis, symptomatic gallbladder disease (excluding cholecystectomy), history of pancreatic injury, or other factors that may lead to a high risk of pancreatitis; |
| | 11) History of gastrointestinal diseases such as gastroparesis, esophageal motility disorders, gastroesophageal reflux, chronic diarrhea, fecal incontinence, and constipation, which in the investigator's opinion make the subject unsuitable for participation in this study. |
| | 8. Any laboratory test result meeting any of the following criteria at screening: |
| | 1) Serum calcitonin ≥ 50 ng/L (pg/mL); |
| | 2) Alanine aminotransferase (ALT) ≥ 3.0 × upper limit of normal (ULN) and/or aspartate aminotransferase (AST) ≥ 3.0 × ULN and/or total bilirubin ≥ 2.0 × ULN; |
| | 3) Estimated glomerular filtration rate (eGFR) < 60 mL/min/1.73 m², calculated using the CKD-EPI formula; |
| | 4) Thyroid dysfunction (TSH > 6 mIU/L or < 0.4 mIU/L); |
| | 5) Fasting triglycerides ≥ 5.64 mmol/L (500 mg/dL); |
| | 6) Serum amylase or lipase > 1.5 × ULN; |
| | 7) International normalized ratio (INR) for prothrombin time exceeding the upper limit of the normal range; |
| | 8) Hemoglobin < 110 g/L (males) or < 100 g/L (females); |
| | 9) Fasting venous plasma glucose ≥ 7.0 mmol/L or 2-hour venous plasma glucose ≥ 11.1 mmol/L after a 75 g oral glucose tolerance test (OGTT); |
| | 10) Positive for human immunodeficiency virus (HIV) antibody, positive for *Treponema pallidum* antibody, positive for hepatitis B surface antigen (HBsAg), or positive for hepatitis C virus (HCV) antibody. |
| | 9. 12-lead electrocardiogram (ECG) findings with clinical significance at screening: heart rate < 50 bpm or > 100 bpm, second- or third-degree atrioventricular block, long QT syndrome or QTc > 500 ms, left or right bundle branch block, Wolff-Parkinson-White syndrome, or other significant arrhythmias (excluding sinus arrhythmia). |
| | 10. Blood donation and/or blood loss ≥ 400 mL or bone marrow donation within 3 months prior to screening, or presence of hematological disorders (including but not limited to hemoglobinopathies, hemolytic anemia, thalassemia, sickle cell anemia). |
| | 11. Undergone bariatric surgery (excluding acupuncture for weight loss, liposuction, and abdominoplasty) within 1 year prior to screening. |
| | 12. History of organ transplantation; or major or moderate surgery, severe trauma, or severe infection within 6 months prior to screening, which in the investigator's opinion makes the subject unsuitable for participation; or planned surgery during the trial period, excluding outpatient surgeries deemed by the investigator to have no impact on subject safety or trial results. |
| | 13. Use of any of the following medications or treatments within 3 months prior to screening: |
| | 1) Use of GLP-1 receptor (GLP-1R) agonists, GLP-1R/glucagon receptor (GCGR) agonists, gastric inhibitory polypeptide receptor (GIPR)/GLP-1R agonists, or GIPR/GLP-1R/GCGR agonists. |
| | 2) Use of medications affecting body weight, including systemic corticosteroids (intravenous, oral, or intra-articular), selective serotonin reuptake inhibitors (SSRIs), serotonin-norepinephrine reuptake inhibitors (SNRIs), tricyclic/tetracyclic antidepressants, other psychiatric medications, or sedatives (e.g., imipramine, amitriptyline, mirtazapine, paroxetine, phenelzine, chlorpromazine, thioridazine, clozapine, olanzapine, valproic acid, valproate derivatives, lithium salts), diuretics, etc. |
| | 3) Use of traditional Chinese medicines, proprietary Chinese medicines, herbal remedies, health supplements, meal replacements, etc., that affect body weight. |
| | 4) Use of weight-loss medications, such as: sibutramine hydrochloride, orlistat, phentermine, phenylpropanolamine, mazindol, phentermine, amfepramone, lorcaserin, phentermine/topiramate combination, naltrexone/bupropion combination, etc., either previously or currently. |
| | 5) Use of glucose-lowering medications, such as metformin, alpha-glucosidase inhibitors, sulfonylureas, DPP-4 inhibitors, SGLT-2 inhibitors, thiazolidinediones (TZDs), etc. |
| | 14. Participation in any clinical study of an investigational drug, vaccine, or medical device, and having received treatment, within 3 months prior to screening. |
| | 15. Any other condition that, in the opinion of the investigator, may affect the evaluation of efficacy or safety in this study, making the subject unsuitable for participation. |

### Statistical Methods:

### Analysis Sets

FAS (Full Analysis Set): Includes all randomized subjects who received at least one dose of the investigational product. This dataset serves as the primary analysis set for demographics, baseline characteristics, medical history, and efficacy analyses.

PPS (Per Protocol Set): A subset of the FAS, comprising subjects who met all inclusion criteria, did not meet any exclusion criteria, had a primary endpoint efficacy measurement, and had no other major protocol deviations. This dataset is used for supportive analyses of the primary efficacy endpoint.

SS (Safety Set): Includes all randomized subjects who received at least one dose of the investigational product and have at least one safety assessment. This dataset is used for the statistical analysis of safety indicators.

PKCS (Pharmacokinetic Concentration Set): Includes all randomized subjects who received at least one dose of the investigational product and have at least one measurable post-dose drug concentration.

mPKCS (intensive PKCS): A subset of the PKCS, including all randomized subjects who received at least one dose of the investigational product, underwent intensive blood sampling, and have at least one measurable drug concentration from the intensive sampling.

PKPS (Pharmacokinetic Parameter Set): Includes all randomized subjects who received at least one dose of the investigational product and have at least one valid post-dose PK parameter.

mPKPS (intensive PKPS): A subset of the PKPS, including all randomized subjects who received at least one dose of the investigational product, underwent intensive blood sampling, and have at least one valid PK parameter from the intensive sampling.

### General Methods

For the GZR18 48 mg once every two weeks (Q2W) dose group, due to the actual clinical trial conduct, some subjects received a maintenance dose of 36 mg during the stable dose period and did not reach the 48 mg dose level. Therefore, this dose group was split into "GZR18 36 mg Q2W", "GZR18 48 mg Q2W", and "Other Dose Groups" for additional statistical analyses against the pooled placebo, aiming to further explore the efficacy of the GZR18 36 mg Q2W dose group. Efficacy indicators analyzed included body weight, BMI, waist circumference, hip circumference, and waist-to-hip ratio. Splitting rule: subjects with a Week 28 dose of 48 mg were assigned to the "GZR18 48 mg Q2W" group; subjects with a Week 28 dose of 36 mg were assigned to the "GZR18 36 mg Q2W" group; subjects with a Week 28 dose other than 48 mg or 36 mg, or who were not dosed, were assigned to the "Other Dosage Groups".

### Efficacy Analysis

### Primary Estimand Analysis

Missing body weight data were imputed using the SAS procedure PROC MI with the Fully Conditional Specification (FCS) method, based on the Full Analysis Set (FAS).

Primary Analysis Model: Based on the FAS, an analysis of covariance (ANCOVA) was performed on the percentage change in body weight from baseline to Week 30. The percentage change from baseline in body weight was the dependent variable, baseline body weight was included as a covariate, and treatment group was a fixed effect. In the models comparing GZR18 24 mg (or 18 mg) once weekly with placebo, and GZR18 48 mg (or 36 mg) once every two weeks with placebo, adherence to the planned stable dose period was added as an additional covariate. The least squares (LS) means and their two-sided 95% confidence intervals (CIs) for the percentage change from baseline were calculated for each treatment group, along with the between-group differences in LS means (GZR18 - placebo) and their two-sided 95% CIs.

The analysis results after imputation were combined and summarized using the SAS procedure PROC MIANALYZE. If the upper limit of the two-sided 95% CI for the combined between-group difference in LS means (GZR18-placebo) was less than 0, and the one-sided p-value for the between-group comparison was < 0.025, this indicated that GZR18 was superior to placebo.

### Secondary Estimand Analysis

### Continuous Variables

Primary Analysis Method: Based on the FAS, missing data were imputed using multiple imputation. The imputed data were analyzed using the same analytical methods as those employed for the primary estimand analysis. Finally, the analysis results were combined across all visits.

### Binary Variables

### 1) Proportion of subjects achieving ≥5%, 10%, 15%, 20%, and 25% reduction in body weight from baseline at each visit.

Primary Analysis Method: Missing data at each visit were imputed using an imputation method consistent with the primary analysis method for the percentage change from baseline in body weight at each visit in the secondary estimand. Binary variables were then derived. Statistical analysis on the imputed data was performed using a generalized linear model (logistic regression model for some analyses), with baseline body weight as a covariate and treatment group as a fixed effect. The between-group rate difference and its two-sided 95% CI were calculated.

### 2) Proportion of subjects achieving ≥5% reduction in body weight from baseline at Week 30.

Primary Analysis Method: Based on the FAS, missing data at Week 30 were imputed using an imputation method consistent with that used for the primary analysis method in the primary estimand. Binary variables were then derived. Statistical analysis on the imputed data was performed using a generalized linear model (logistic regression model for some analyses), with baseline body weight as a covariate and treatment group as a fixed effect. The between-group rate difference and its two-sided 95% CI were calculated.

### Study Results:

### 1. Subject Disposition

A total of 465 subjects were screened in this study, of whom 340 (73.1%) were successfully screened and 125 (26.9%) failed screening. Reasons for screening failure were: 121 (26.0%) "did not meet inclusion criteria/meet exclusion criteria", two (0.4%) were "subject withdrew informed consent", and two were (0.4%) "other reasons".

A total of 340 subjects were randomized in this study, including 52 subjects in the Compound (I) 12 mg (Q2W) group, 53 subjects in the Compound (I) 18 mg (Q2W) group, 52 subjects in the Compound (I) 24 mg (Q2W) group, 64 subjects in the Compound (I) 48 mg (Q2W) group, 53 subjects in the Compound (I) 24 mg (18 mg) (QW) group, and 66 subjects in the placebo group. Among all randomized subjects, 340 (100.0%) received the investigational product.

A total of 286 (84.1%) subjects completed the study, including 44 (84.6%) in the Compound (I) 12 mg (Q2W) group, 49 (92.5%) in the Compound (I) 18 mg (Q2W) group, 45 (86.5%) in the Compound (I) 24 mg (Q2W) group, 46 (71.9%) in the Compound (I) 48 mg (Q2W) group, 45 (84.9%) in the Compound (I) 24 mg (18 mg) (QW) group, and 57 (86.4%) in the placebo group.

### 2. Demographics and Baseline Characteristics

A total of 340 subjects were enrolled in the study. The mean age ± standard deviation (SD) of the subjects was 33.1 ± 7.23 years; there were 185 (54.4%) males and 155 (45.6%) females; 326 (95.9%) were Han Chinese and 14 (4.1%) were of other ethnicities; mean height ± SD was 169.00 ± 9.418 cm; mean body weight ± SD was 95.63 ± 19.712 kg; mean BMI ± SD was 33.24 ± 4.621 kg/m², with 313 (92.1%) being obese (≥ 28 kg/m²) and 27 (7.9%) being overweight (≥ 24 and < 28 kg/m²); mean waist circumference ± SD was 106.81 ± 12.541 cm; mean hip circumference ±SD was 112.33 ± 9.335 cm; mean waist-to-hip ratio ± SD was 0.95 ± 0.074.

Demographics and baseline characteristics were generally balanced and comparable across treatment groups.

### 3. Treatment Compliance

In the study, 332 (97.6%) subjects had prescribed dose compliance between 80% and 120%, and 8 (2.4%) subjects had prescribed dose compliance < 80%.

In the study, 300 (88.2%) subjects had scheduled dose compliance between 80% and 120%, and 40 (11.8%) subjects had scheduled dose compliance < 80%.

All 340 (100.0%) subjects were included in the Full Analysis Set (FAS) and Safety Set (SS); 274 (80.6%) subjects were included in the Per Protocol Set (PPS); 250 (73.5%) subjects were included in the Pharmacokinetic Concentration Set (PKCS); 79 (23.2%) subjects were included in the intensive Pharmacokinetic Concentration Set (mPKCS), Pharmacokinetic Parameter Set (PKPS), and intensive Pharmacokinetic Parameter Set (mPKPS).

Overweight or obese subjects received subcutaneous injections of Compound (I) Injection at four different target doses administered once every two weeks (12 mg, 18 mg, 24 mg, 48 mg) and one target dose administered once weekly (18 mg/24 mg) for a 30-week period. The results showed that Compound (I) Injection effectively reduced body weight in overweight or obese subjects, with a trend towards improvement in metabolic parameters such as waist circumference, blood glucose, blood lipids, blood pressure, uric acid, and liver enzymes.

### 4. Efficacy Results

### Body Weight Change Parameters

The primary efficacy endpoint of this study was the percentage change in body weight from baseline at the end of the study, Week 30 (W30). The primary endpoint, percentage change in body weight from baseline at W30, was tested using a fixed-sequence testing procedure. The testing order was as follows:
1) Compound (I) - 48 mg Q2W vs. Placebo
2) Compound (I) - 24 mg (or 18 mg) QW vs. Placebo
3) Compound (I) - 24 mg Q2W vs. Placebo
4) Compound (I) - 18 mg Q2W vs. Placebo
5) Compound (I) - 12 mg Q2W vs. Placebo

The significance level was one-sided α = 0.025. Testing would proceed to the next dose group only if the p-value for the preceding dose group was < 0.025, indicating statistical significance. Testing was stopped for any dose group where the p-value was ≥ 0.025. The primary analysis method was based on the FAS. Multiple imputation was performed for each dose group using subjects in the pooled placebo group with non-missing W30 data; the placebo group was imputed based on its own data. Imputation variables included baseline body weight, W30 body weight, baseline BMI category (obese: BMI ≥ 28 kg/m², overweight: 24 kg/m² ≤ BMI < 28 kg/m²), and sex.

**Table 3: Summary of Change from Baseline in Body Weight at W30 (kg) (Primary Analysis Method: LR MI1 (FAS))**

| Testing Sequence | Dose Groups | Time Point Statistics | Compound (I) | Placebo (**N=66)** |
|---|---|---|---|---|
| 1 | Compound (I) 48mg (once every two weeks) (N=64) | Percentage change from baseline at W30 | | |
| | | n (missing data) | 46(18) | 55(11) |
| | | Mean (SD) | -17.44 (6.977) | -0.84 (6.230) |
| | | Median | -17.26 | 0.00 |
| | | Q1, Q3 | -23.33, -12.10 | -3.03, 2.60 |
| | | Min, Max | -33.0, -2.7 | -25.1, 9.2 |
| | | LSMean (SE) | -16.22(1.102) | -0.71(1.070) |
| | | 95%CI | (-18.38,-14.06) | (-2.81,1.39) |
| | | LSMean between-group difference -vs. Placebo (SE) | -15.51(1.590) | |
| | | 95%CI | (-18.62,-12.39) | |
| | | One-sided p-value for between-group comparison | <0.001 | |
| 2 | Compound (I) 24mg (18mg) (once weekly) (N=53) | Percentage change from baseline at W30 | | |
| | | n (missing data) | 45(8) | 55(11) |
| | | Mean (SD) | -17.74 (6.523) | -0.84 (6.230) |
| | | Median | -17.88 | 0.00 |
| | | Q1, Q3 | -21.62, -13.56 | -3.03, 2.60 |
| | | Min, Max | -33.6, -1.8 | -25.1, 9.2 |
| | | LSMean (SE) | -17.19(1.006) | -0.50(0.972) |
| | | 95%CI | (-19.16,-15.22) | (-2.41,1.41) |
| | | LSMean between-group difference -vs. Placebo (SE) | -16.69(1.423) | |
| | | 95%CI | (-19.48,-13.90) | |
| | | One-sided p-value for between-group comparison | <0.001 | |
| 3 | Compound (I) 24mg (once every two weeks) (N=52) | compound (I) 24mg (every two weeks) (N=52) | | |
| | | n (missing) | 45(7) | 55(11) |
| | | Mean (SD) | -14.41 (5.917) | -0.84 (6.230) |
| | | Median | -14.74 | 0.00 |
| | | Q1, Q3 | -17.79, -10.60 | -3.03, 2.60 |
| | | Min, Max | -27.0, -0.9 | -25.1, 9.2 |
| | | LSMean (SE) | -12.61(1.020) | -0.82(0.951) |
| | | 95%CI | (-14.61,-10.60) | (-2.69,1.05) |
| | | LSMean between-group difference -vs. Placebo (SE) | -11.79(1.438) | |
| | | 95%CI | (-14.61,-8.96) | |
| | | One-sided p-value for between-group comparison | <0.001 | |
| 4 | Compound (I) 18mg (once every two weeks) (N=53) | Percentage change from baseline at W30 | | |
| | | n (missing data) | 49(4) | 55(11) |
| | | Mean (SD) | -13.23 (6.471) | -0.84 (6.230) |
| | | Median | -12.95 | 0.00 |
| | | Q1, Q3 | -18.64, -8.34 | -3.03, 2.60 |
| | | Min, Max | -30.0, -3.1 | -25.1, 9.2 |
| | | LSMean (SE) | -12.22(1.003) | -0.79(0.965) |
| | | 95%CI | (-14.19,-10.25) | (-2.69, 1.10) |
| | | LSMean between-group difference -vs. Placebo (SE) | -11.43(1.396) | |
| | | 95%CI | (-14.17,-8.69) | |
| | | One-sided p-value for between-group comparison | <0.001 | |
| 5 | Compound (I) 12mg (once every two weeks) (N=52) | Percentage change from baseline at W30 | | |
| | | n (missing) | 44(8) | 55(11) |
| | | Mean (SD) | -10.76 (7.112) | -0.84 (6.230) |
| | | Median | -10.75 | 0.00 |
| | | Q1, Q3 | -15.46, -5.42 | -3.03, 2.60 |
| | | Min, Max | -29.5, 1.0 | -25.1, 9.2 |
| | | LSMean (SE) | -9.27(1.070) | -0.82(0.974) |
| | | 95%CI | (-11.37,-7.17) | (-2.73,1.09) |
| | | LS Mean between-group difference -vs. Placebo (SE) | -8.46(1.393) | |
| | | 95%CI | (-11.19,-5.72) | |
| | | One-sided p-value for between-group comparison | <0.001 | |

The results demonstrated that for the Compound (I) 48 mg (Q2W) group, the Compound (I) 24 mg (or 18 mg) (QW) group, the Compound (I) 24 mg (Q2W) group, the Compound (I) 18 mg (Q2W) group, and the Compound (I) 12 mg (Q2W) group, the Least Squares (LS) mean differences in body weight change from baseline compared to the placebo group were -14.83 kg [95% CI: (-17.84, -11.81)], -15.82 kg [95% CI: (-18.48, -13.16)], -11.09 kg [95% CI: (-13.72, -8.47)], -10.70 kg [95% CI: (-13.15, -8.24)], and -7.91 kg [95% CI: (-10.43, -5.40)], respectively. The LS mean differences in percentage change from baseline were -15.51% [95% CI: (-18.62%, -12.39%)], -16.69% [95% CI: (-19.48%, -13.90%)], -11.79% [95% CI: (-14.61%, -8.96%)], -11.43% [95% CI: (-14.17%, -8.69%)], and -8.46% [95% CI: (-11.19%, -5.72%)], respectively. Tested according to the fixed-sequence procedure described above, the one-sided p-values for the between-group differences comparing each dose group to placebo were all < 0.001, demonstrating that the percentage change in body weight from baseline at W30 was superior to placebo for all dose groups.Proportion of Subjects Achieving ≥5%, ≥10%, ≥15%, ≥20%, and ≥25% Reduction in Body Weight from Baseline

The primary analysis method was based on the FAS, using multiple imputation with modeling based on the placebo group. Logistic regression models were used to calculate the 95% confidence intervals (CIs) for each group and the adjusted between-group rate differences. The bootstrap method was used to calculate the standard error and 95% CI for the rate differences. The percentages presented are the results after multiple imputation, analyzed using logistic regression models, and finally pooled and summarized using Rubin's rules, as shown in table 4.

**Table 4: Proportion of Subjects Achieving ≥5%, ≥10%, ≥15%, ≥20%, and ≥25% Reduction in Body Weight from Baseline at W30**

| **Primary Analysis Method: LR MI1 (FAS)** | | | | | | |
|---|---|---|---|---|---|---|
| **Time point Statistical parameter** (W30) | **Compound (I) (once every two weeks)** | | | | **Compound (I) (once weekly)** | Placebo **(N=66)** |
| | **12mg (N=52)** | **18mg (N=53)** | **24mg (N=52)** | **48mg (N=64)** | **24mg (18mg) (N=53)** | |
| Proportion of subjects with ≥5% reduction in body weight from baseline | | | | | | |
| N(%) | 37(71.8) | 45(86.4) | 46(88.5) | 60(93.9) | 48(91.0) | 12(18.4) |
| 95% CI | 67.2, 76.3 | 83.2, 89.7 | 85.0, 92.1 | 89.8, 98.1 | 87.4, 94.7 | 15.6, 21.1 |
| Compound (I)-placebo | | | | | | |
| RD (95% CI) | 54.5(38.5, 70.5) | 67.9(54.3, 81.5) | 73.7(62.1, 85.4) | 78.5(68.1, 88.9) | 73.5(61.0, 86.0) | |
| Proportion of subjects with ≥10% reduction in body weight from baseline | | | | | | |
| N(%) | 25(48.7) | 32(61.0) | 37(72.1) | 49(77.4) | 45(85.5) | 4(6.7) |
| 95% CI | 45.2, 52.2 | 58.9, 63.1 | 68.4, 75.9 | 72.7, 82.2 | 82.1, 89.0 | 4.9, 8.5 |
| Compound (I)-placebo | | | | | | |
| RD (95% CI) | 38.7(25.8, 51.6) | 54.1(40.7, 67.5) | 63.6(48.1, 79.0) | 70.3(57.5, 83.0) | 76.9(64.6, 89.2) | |
| Proportion of subjects with ≥15% reduction in body weight from baseline | | | | | | |
| N (%) | 12(23.8) | 19(35.5) | 20(40.9) | 35(55.1) | 32(61.0) | 2(4.0) |
| 95% CI | 21.9, 25.6 | 33.9, 37.1 | 38.7, 43.1 | 50.3, 60.0 | 58.1, 63.9 | 2.3, 5.6 |
| Compound (I)-placebo | | | | | | |
| RD (95% CI) | 18.6(6.0, 31.2) | 31.0(18.6, 43.3) | 36.1(20.6, 51.7) | 51.5(37.5, 65.5) | 57.3(44.0, 70.6) | |
| Proportion of subjects with ≥20% reduction in body weight from baseline | | | | | | |
| N(%) | 4(7.9) | 9(16.6) | 9(18.6) | 20(32.1) | 19(36.0) | 2(3.3) |

| **Time point Statistical parameter** (W30) | **Compound (I) (once every two weeks)** | | | | **Compound (I) (once weekly)** | Placebo **(N=66)** |
|---|---|---|---|---|---|---|
| | **12mg (N=52)** | **18mg (N=53)** | **24mg (N=52)** | **48mg (N=64)** | **24mg (18mg) (N=53)** | |
| 95% CI | 7.1, 8.7 | 15.8, 17.4 | 16.6, 20.5 | 28.1, 36.1 | 33.2, 38.8 | 2.0, 4.6 |
| Compound (I)-placebo | | | | | | |
| RD (95% CI) | 4.7(-5.6, 15.0) | 13.6(3.9, 23.3) | 14.8(4.0, 25.6) | 28.0(15.5, 40.5) | 32.4(18.3, 46.6) | |
| Proportion of subjects with ≥25% reduction in body weight from baseline | | | | | | |
| N (%) | 2(3.9) | 1(1.9) | 1(2.0) | 4(7.4) | 5(10.1) | 1(1.6) |
| 95% CI | 3.3, 4.5 | 1.8, 1.9 | 1.4, 2.5 | 5.1, 9.8 | 7.6, 12.6 | 1.0, 2.2 |
| Compound (I)-placebo | | | | | | |
| RD (95% CI) | 2.3(-4.3, 9.0) | 0.3(-4.2, 4.9) | 0.6(-5.2, 6.3) | 4.7(-2.5, 11.9) | 8.1(-1.0, 17.2) | |

Conclusion: As shown in table 4, the proportions of subjects achieving a ≥5% reduction in body weight from baseline at W30 were 71.8%, 86.4%, 88.5%, 93.9%, 91.0%, and 18.4% in the Compound (I) 12 mg (Q2W) group, Compound (I) 18 mg (Q2W) group, Compound (I) 24 mg (Q2W) group, Compound (I) 48 mg (Q2W) group, Compound (I) 24 mg (18 mg) (QW) group, and placebo group, respectively. The rate differences versus the placebo group for each respective group were 54.5% [95% CI: (38.5%, 70.5%)], 67.9% [95% CI: (54.3%, 81.5%)], 73.7% [95% CI: (62.1%, 85.4%)], 78.5% [95% CI: (68.1%, 88.9%)], and 73.5% [95% CI: (61.0%, 86.0%)], respectively.

The proportions of subjects achieving a ≥10% reduction in body weight from baseline at W30 were 48.7%, 61.0%, 72.1%, 77.4%, 85.5%, and 6.7% in the Compound (I) 12 mg (Q2W) group, Compound (I) 18 mg (Q2W) group, Compound (I) 24 mg (Q2W) group, Compound (I) 48 mg (Q2W) group, Compound (I) 24 mg (18 mg) (QW) group, and placebo group, respectively. The rate differences versus the placebo group for each respective group were 38.7% [95% CI: (25.8%, 51.6%)], 54.1% [95% CI: (40.7%, 67.5%)], 63.6% [95% CI: (48.1%, 79.0%)], 70.3% [95% CI: (57.5%, 83.0%)], and 76.9% [95% CI: (64.6%, 89.2%)].

The proportions of subjects achieving a ≥15% reduction in body weight from baseline at W30 were 23.8%, 35.5%, 40.9%, 55.1%, 61.0%, and 4.0% in the Compound (I) 12 mg (Q2W) group, Compound (I) 18 mg (Q2W) group, Compound (I) 24 mg (Q2W) group, Compound (I) 48 mg (Q2W) group, Compound (I) 24 mg (18 mg) (QW) group, and placebo group, respectively. The rate differences versus the placebo group for each respective group were 18.6% [95% CI: (6.0%, 31.2%)], 31.0% [95% CI: (18.6%, 43.3%)], 36.1% [95% CI: (20.6%, 51.7%)], 51.5% [95% CI: (37.5%, 65.5%)], and 57.3% [95% CI: (44.0%, 70.6%)].

The proportions of subjects achieving a ≥20% reduction in body weight from baseline at W30 were 7.9%, 16.6%, 18.6%, 32.1%, 36.0%, and 3.3% in the Compound (I) 12 mg (Q2W) group, Compound (I) 18 mg (Q2W) group, Compound (I) 24 mg (Q2W) group, Compound (I) 48 mg (Q2W) group, Compound (I) 24 mg (18 mg) (QW) group, and placebo group, respectively. The rate differences versus the placebo group for each respective group were 4.7% [95% CI: (-5.6%, 15.0%)], 13.6% [95% CI: (3.9%, 23.3%)], 14.8% [95% CI: (4.0%, 25.6%)], 28.0% [95% CI: (15.5%, 40.5%)], and 32.4% [95% CI: (18.3%, 46.6%)].

The proportions of subjects achieving a ≥25% reduction in body weight from baseline at W30 were 3.9%, 1.9%, 2.0%, 7.4%, 10.1%, and 1.6% in the Compound (I) 12 mg (Q2W) group, Compound (I) 18 mg (Q2W) group, Compound (I) 24 mg (Q2W) group, Compound (I) 48 mg (Q2W) group, Compound (I) 24 mg (18 mg) (QW) group, and placebo group, respectively. The rate differences versus the placebo group for each respective group were 2.3% [95% CI: (-4.3%, 9.0%)], 0.3% [95% CI: (-4.2%, 4.9%)], 0.6% [95% CI: (-5.2%, 6.3%)], 4.7% [95% CI: (-2.5%, 11.9%)], and 8.1% [95% CI: (-1.0%, 17.2%)].

The reductions in body weight from baseline in all Compound (I) dose groups were statistically significant compared to the placebo group, demonstrating that the weight loss efficacy in all Compound (I) dose groups was superior to that of the placebo group.

In the Compound (I) Q2W groups, the proportions of subjects achieving ≥5%, 10%, 15%, and 20% reduction in body weight from baseline increased with increasing dose.

### Change from Baseline in BMI

The primary analysis method was based on the FAS. Missing data were imputed using multiple imputation under the Missing at Random (MAR) assumption. LS Means were derived from an ANCOVA model, with the change from baseline or percentage change from baseline as the dependent variable, baseline value as a covariate, and treatment group as a fixed effect.

Summary of Change from Baseline in BMI at W30 (kg/m²) - Primary Analysis Method: ANCOVA MI (FAS). See Table 5 for details.

**Table 5: Summary of Change from Baseline in BMI at W30 (kg/m²)**

| Primary Analysis Method: ANCOVA MI (FAS) | | | | | | |
|---|---|---|---|---|---|---|
| **Time point** Statistics | **GZR18**(**every two weeks**) | | | | **GZR18**(**once weekly**) | Placebo **(N=66)** |
| | **12mg (N=52)** | **18mg (N=53)** | **24mg (N=52)** | **48mg (N=64)** | **24mg (18mg) (N=53)** | |
| Change from Baseline at W30 | | | | | | |
| n (missing) | 44(8) | 49(4) | 45(7) | 46(18) | 45(8) | 55(11) |
| Mean (SD) | -3.63 (2.408) | -4.28 (2.130) | -4.72 (1.831) | -5.76 (2.434) | -5.86 (2.400) | -0.31 (2.032) |
| Median | -3.65 | -4.10 | -4.90 | -5.90 | -5.80 | 0.00 |
| Q1, Q3 | -5.05, -1.75 | -5.60, -2.60 | -6.10, -3.70 | -7.50, -3.80 | -6.70, -4.40 | -1.20, 0.90 |
| Min, Max | -11.4, 0.3 | -9.2, -1.0 | -8.2, -0.3 | -11.1, -1.0 | -11.6, -0.6 | -8.3, 2.9 |
| LSMean(SE) | -3.39(0.333) | -4.18(0.317) | -4.56(0.332) | -5.78(0.322) | -5.68(0.320) | -0.32(0.292 ) |
| 95%CI | (-4.04,-2.73) | (-4.80,-3.56) | (-5.21,-3.91) | (-6.41,-5.15) | (-6.31,-5.06) | (-0.89,0.25) |

| **Time point** Statistics | **GZR18 (every two weeks)** | | | | **GZR18 (once weekly)** | Placebo **(N=66)** |
|---|---|---|---|---|---|---|
| | **12mg (N=52)** | **18mg (N=53)** | **24mg (N=52)** | **48mg (N=64)** | **24mg (18mg) (N=53)** | |
| LSMean between-group difference -vs. Placebo (SE) | -3.07(0.442) | -3.86(0.432) | -4.24(0.439) | -5.46(0.439) | -5.36(0.431) | |
| 95%CI | (-3.93,-2.20) | (-4.71,-3.01) | (-5.10,-3.38) | (-6.32,-4.60) | (-6.21,-4.52) | |

| Percentage Change from Baseline at W30 | | | | | | |
|---|---|---|---|---|---|---|
| n (missing) | 44(8) | 49(4) | 45(7) | 46(18) | 45(8) | 55(11) |
| Mean (SD) | -10.80 (7.097) | -13.22 (6.472) | -14.40 (5.901) | -17.34 (7.069) | -17.72 (6.533) | -0.81 (6.250) |
| Median | -10.93 | -13.11 | -14.54 | -17.40 | -17.94 | 0.00 |
| Q1, Q3 | -15.48, -5.59 | -18.64, -8.36 | -17.66, -10.63 | -23.41, -12.16 | -21.74, -13.67 | -3.04, 2.75 |
| Min, Max | -29.7, 1.0 | -29.9, -3.0 | -26.9, -0.8 | -32.9, -2.7 | -33.4, -1.7 | -25.1, 9.5 |
| LSMean (SE) | -10.27(1.025) | -12.66(0.967) | -13.89(1.008 ) | -17.57(0.981) | -17.17(0.978) | -1.03(0.888 ) |
| 95%CI | (-12.28,-8.26) | (-14.55,-10.76 ) | (-15.86,-11.9 1) | (-19.49,-15.64 ) | (-19.09,-15.25) | (-2.77,0.71) |
| LSMean between-group difference -vs. Placebo (SE) | -9.25(1.352) | -11.63(1.316) | -12.86(1.335 ) | -16.54(1.335) | -16.14(1.313) | |
| 95%CI | (-11.90,-6.59) | (-14.21,-9.05) | (-15.48,-10.2 4) | (-19.16,-13.92 ) | (-18.72,-13.57) | |

Based on the data above, it can be seen that the between-group differences in LS means for change from baseline in BMI at W30 versus the placebo group were -3.07 kg/m² [95% CI: (-3.93, -2.20)], -3.86 kg/m² [95% CI: (-4.71, -3.01)], -4.24 kg/m² [95% CI: (-5.10, -3.38)], -5.46 kg/m² [95% CI: (-6.32, -4.60)], and -5.36 kg/m² [95% CI: (-6.21, -4.52)] for the Compound (I) 12 mg (Q2W) group, Compound (I) 18 mg (Q2W) group, Compound (I) 24 mg (Q2W) group, Compound (I) 48 mg (Q2W) group, and Compound (I) 24 mg (18 mg) (QW) group, respectively.

In both the FAS and PPS, all Compound (I) dose groups showed greater reductions in BMI from baseline at W30 Compoundcompared to the placebo group. In the Compound (I) Q2W groups, the magnitude of BMI reduction increased with increasing dose. The results were consistent across various analytical methods.

### Change from Baseline in Hip Circumference

The primary analysis method was based on the FAS. Missing data were imputed using multiple imputation under the MAR assumption. LS Means were derived from an ANCOVA model, with the change from baseline or percentage change from baseline as the dependent variable, baseline value as a covariate, and treatment group as a fixed effect.

Summary of Change from Baseline in Hip Circumference at W30 (cm) - Primary Analysis Method: ANCOVA MI (FAS). See Table 6 for details.

**Table 6: Summary of Change from Baseline in Hip Circumference at W30 (cm)**

| **Primary Analysis Method: ANCOVA MI (FAS)** | | | | | | |
|---|---|---|---|---|---|---|
| **Time point** Statistics | **GZR18 (every two weeks)** | | | | **GZR18 (once weekly)** | Placebo **(N=66)** |
| | **12mg (N=52)** | **18mg (N=53)** | **24mg (N=52)** | **48mg (N=64)** | **24mg (18mg) (N=53)** | |
| Percentage Change from Baseline at W30 | | | | | | |
| n (missing date) | 44(8) | 49(4) | 45(7) | 46(18) | 45(8) | 55(11) |
| Mean (SD) | -6.66 (5.223) | -7.34 (3.931) | -7.70 (3.964) | -10.81 (5.143) | -10.18 (4.544) | -0.43 (4.004) |
| Median | -6.90 | -6.90 | -7.50 | -9.65 | -9.80 | -0.30 |
| Q1, Q3 | -10.35, -3.35 | -10.30, -4.50 | -10.60, -5.00 | -14.70, -7.50 | -13.40, -8.50 | -2.50, 3.10 |
| Min, Max | -20.2, 5.3 | -17.1, 0.7 | -18.6, -0.4 | -24.3, -0.6 | -21.3, 0.4 | -10.7, 6.4 |
| LSMean (SE) | -6.00(0.721) | -7.16(0.674) | -7.49(0.695) | -10.55(0.707) | -9.77(0.709) | -0.65(0.618 ) |
| 95%CI | (-7.42,-4.59) | (-8.48,-5.84) | (-8.85,-6.12) | (-11.95,-9.16) | (-11.16,-8.38) | (-1.86,0.56) |
| LSMean between-group difference -vs. Placebo (SE) | -5.35(0.942) | -6.51(0.913) | -6.84(0.934) | -9.90(0.941) | -9.12(0.944) | |
| 95%CI | (-7.20,-3.50) | (-8.30,-4.72) | (-8.67,-5.00) | (-11.75,-8.05) | (-10.97,-7.27) | |

| Percentage Change from Baseline at W30 | | | | | | |
|---|---|---|---|---|---|---|
| n (missing data) | 44(8) | 49(4) | 45(7) | 46(18) | 45(8) | 55(11) |
| Mean (SD) | -5.84 (4.563) | -6.61 (3.629) | -6.90 (3.629) | -9.43 (4.118) | -9.07 (3.913) | -0.33 (3.623) |
| Median | -5.60 | -6.07 | -6.61 | -9.33 | -8.58 | -0.29 |
| Q1, Q3 | -8.68, -2.95 | -9.20, -3.83 | -9.50, -4.24 | -12.65, -6.44 | -11.73, -7.83 | -2.08, 2.62 |
| Min, Max | -16.0, 4.6 | -15.8, 0.7 | -16.9, -0.4 | -18.9, -0.5 | -17.8, 0.3 | -10.0, 5.7 |
| LSMean (SE) | -5.33(0.645) | -6.39(0.603) | -6.69(0.617) | -9.33(0.632) | -8.73(0.636) | -0.56(0.550 ) |
| 95%CI | (-6.60,-4.07) | (-7.57,-5.20) | (-7.90,-5.48) | (-10.57,-8.09) | (-9.98,-7.48) | (-1.64,0.52) |
| LS Mean between-group difference -vs. Placebo SE) | -4.77(0.840) | -5.82(0.814) | -6.13(0.831) | -8.77(0.840) | -8.17(0.845) | |
| 95%CI | (-6.42,-3.12) | (-7.42,-4.23) | (-7.76,-4.50) | (-10.42,-7.12) | (-9.82,-6.51) | |

Conclusion: The between-group differences in LS means for change from baseline in hip circumference at W30 versus the placebo group were -5.35 cm [95% CI: (-7.20, -3.50)], -6.51 cm [95% CI: (-8.30, -4.72)], -6.84 cm [95% CI: (-8.67, -5.00)], -9.90 cm [95% CI: (-11.75, -8.05)], and -9.12 cm [95% CI: (-10.97, -7.27)] for the Compound (I) 12 mg (Q2W) group, Compound (I) 18 mg (Q2W) group, Compound (I) 24 mg (Q2W) group, Compound (I) 48 mg (Q2W) group, and Compound (I) 24 mg (18 mg) (QW) group, respectively.

In both the FAS and PPS, all Compound (I) dose groups showed greater reductions in hip circumference from baseline at W30 compared with the placebo group. In the Compound (I) Q2W group, the magnitude of hip circumference reduction increased with increasing dose. The results were consistent across various analytical methods.

### Change from Baseline in HbA1c

The primary analysis method was based on the FAS. Missing data were imputed using multiple imputation under the MAR assumption. LS Means were derived from an ANCOVA model, with the change from baseline or percentage change from baseline as the dependent variable, baseline value as a covariate, and treatment group as a fixed effect.

Summary of change from baseline in HbA1c at W30 (%) - primary analysis method: ANCOVA MI (FAS) is detailed in Table 7.

**Table 7: Summary of Change from Baseline in HbA1c at W30 (%)**

| **Primary Analysis Method: ANCOVA MI (FAS)** | | | | | | |
|---|---|---|---|---|---|---|
| **Time point** Statistics | **GZR18 (once every two weeks)** | | | | **GZR18 (once weekly)** | Placebo **(N=66)** |
| | **12mg (N=52)** | **18mg (N=53)** | **24mg (N=52)** | **48mg (N=64)** | **24mg (18mg) (N=53)** | |
| Change from Baseline at W30 | | | | | | |
| n (missing data) | 44(8) | 49(4) | 45(7) | 46(18) | 45(8) | 56(10) |
| Mean (SD) | -0.290 (0.3310) | -0.242 (0.2715) | -0.349 (0.3803) | -0.434 (0.2847) | -0.489 (0.4373) | 0.053 (0.2940) |
| Median | -0.400 | -0.200 | -0.300 | -0.400 | -0.400 | 0.050 |
| Q1, Q3 | -0.500, -0.050 | -0.500, -0.100 | -0.500, -0.100 | -0.600, -0.200 | -0.700, -0.200 | -0.100, 0.250 |
| Min, Max | -1.20, 0.60 | -0.80, 0.30 | -1.70, 0.50 | -1.30, 0.00 | -2.10, 0.10 | -0.60, 0.80 |
| LS Mean (SE) | -0.285(0.0437 ) | -0.278(0.0413 ) | -0.333(0.043 8) | -0.448(0.0425 ) | -0.467(0.0472 ) | 0.067(0.0404 ) |
| 95%CI | (-0.371,-0.199 ) | (-0.359,-0.198 ) | (-0.419,-0.24 7) | (-0.531,-0.364 ) | (-0.560,-0.374 ) | (-0.012,0.14 7) |
| LS Mean between-group difference -vs. Placebo (SE) | -0.352(0.0577 ) | -0.346(0.0575 ) | -0.400(0.061 1) | -0.515(0.0620 ) | -0.534(0.0633 ) | |
| 95%CI | (-0.466,-0.239 ) | (-0.459,-0.233 ) | (-0.520,-0.28 0) | (-0.637,-0.393 ) | (-0.659,-0.410 ) | |

| Percentage Change from Baseline at W30 | | | | | | |
|---|---|---|---|---|---|---|
| n (missing data) | 44(8) | 49(4) | 45(7) | 46(18) | 45(8) | 56(10) |

| **Time point** Statistics | **GZR18 (once every two weeks)** | | | | **GZR18 (once weekly)** | Placebo **(N=66)** |
|---|---|---|---|---|---|---|
| | **12mg (N=52)** | **18mg (N=53)** | **24mg (N=52)** | **48mg (N=64)** | **24mg (18mg) (N=53)** | |
| Mean (SD) | -5.058 (5.7571) | -4.187 (4.8197) | -5.967 (6.2873) | -7.741 (4.9094) | -8.325 (6.6292) | 1.025 (5.3007) |
| Median | -6.612 | -4.217 | -5.357 | -6.957 | -7.143 | 0.820 |
| Q1, Q3 | -8.621, -0.847 | -8.319, -1.724 | -8.333, -1.923 | -10.733, -4.000 | -11.475, -3.922 | -1.887, 4.461 |
| Min, Max | -18.18, 11.54 | -12.96, 6.82 | -27.87, 9.26 | -22.81, 0.00 | -30.00, 1.89 | -9.23, 15.69 |
| LS Mean (SE) | -4.996(0.7675 ) | -4.732(0.7297 ) | -5.681(0.780 8) | -7.935(0.7569 ) | -8.004(0.8309 ) | 1.215(0.7116 ) |
| 95%CI | (-6.502,-3.490 ) | (-6.162,-3.301 ) | (-7.214,-4.14 9) | (-9.425,-6.446 ) | (-9.639,-6.369 ) | (-0.183,2.61 3) |
| LSMean between-group difference -vs. Placebo (SE) | -6.211(1.0191 ) | -5.947(1.0140 ) | -6.896(1.086 0) | -9.150(1.0991 ) | -9.219(1.1189 ) | |
| 95%CI | (-8.210,-4.212 ) | (-7.936,-3.958 ) | (-9.030,-4.76 2) | (-11.316,-6.98 4) | (-11.421,-7.01 7) | |

Conclusion: the between-group differences in LS means for change from baseline in HbA1c at W30 versus the placebo group were -0.352% [95% CI: (-0.466%, -0.239%)], -0.346% [95% CI: (-0.459%, -0.233%)], -0.400% [95% CI: (-0.520%, -0.280%)], -0.515% [95% CI: (-0.637%, -0.393%)], and -0.534% [95% CI: (-0.659%, -0.410%)] for the Compound (I) 12 mg (Q2W) group, Compound (I) 18 mg (Q2W) group, Compound (I) 24 mg (Q2W) group, Compound (I) 48 mg (Q2W) group, and Compound (I) 24 mg (18 mg) (QW) group, respectively.

In both the FAS and PPS, all Compound (I) dose groups showed greater reductions in HbA1c from baseline at W30 compared with the placebo group. The results were consistent across various analytical methods. No dose-dependent trend was observed among the Compound (I) Q2W dose groups.

### Example 4: Weight-Loss Effects of Compound (I)

A long-term randomized trial of safety and efficacy was conducted to evaluate weight loss outcomes and other long-term results of compound (I) in 36 (N=36) obesity/overweight adult subjects. Subject inclusion and exclusion criteria are described in Table 2.

Compound (I) injection was manufactured and supplied by Gan & Lee Pharmaceuticals Co., Ltd. Administration: Subcutaneous injection (equilibrate to room temperature before injection). Placebo injection: the placebo consists of the compound (I) solvent excipient, free of Compound (I) and the remaining ingredients are identical to those in compound (I) injection. The placebo has the same appearance, weight, odor, etc as the test drug. It was manufactured and supplied by Gan & Lee Pharmaceuticals Co., Ltd.

Subjects were randomized to receive either compound (I) injection or an equal-volume placebo according to the dosage regimen in Table 8. All drugs were administered using 3-ml disposable pen type syringes. The pen type syringe was used for both the compound (I) injection and placebo administration.

Regardless of diet, the injection shall be administered at any time of day into the thigh, abdomen, or upper arm. During the trial, the injection is administered on the same day of the week.

**Table 8: Dose-Escalation dosage regimen for Compound (I) Injection in Weight Loss Project**

| **Number** | **Week** | **Dosage Design** | | |
|---|---|---|---|---|
| | | **Weekly dose** | **B1 Group** | **B2 Group** |
| 1 | 0 | 1.5 mg | Once a week | Once a week |
| 2 | 1~2 | 3 mg | Once a week | Once a week |
| 3 | 3~6 | 5mg | Once a week | Once a week |
| 4 | 7~10 | 7mg | Once a week | Once a week |
| 5 | 11~14 | 9mg | Once a week | Once every two weeks |
| 6 | 15~18 | 12 mg | Once a week | Once every two weeks |
| 7 | 19~22 | 15mg | Once a week | Once every two weeks |
| 8 | 23~26 | 18mg | Once a week | Once every two weeks |
| 9 | 27~30 | 24 mg | Once a week | Once every two weeks |
| 10 | 31~34 | 30mg | Once a week | Once every two weeks |

The compound (I) injection and an equal volume of placebo were administered to subjects according to the dosage regimen in Table 3. The results of the efficacy and safety assessments for the subjects are shown in Table 9.

**Table 9: Efficacy and Safety of Compound (I) in Treating Overweight or Obese Patients in China***

| | Compound (I) QW** (N=16) | Compound (I) Q2W** (N=10) | Placebo (N=9) |
|---|---|---|---|
| **Demographics** | | | |
| Age (years) † | 35.2(8.8) | | 40.1(9.9) |
| Male, n(%) | 12(46.2) | | 5(55.6) |

| **Weight (kg)** | | | |
|---|---|---|---|
| Baseline† | 91.8(15.1) | 100.4(22.5) | 94.7(16.5) |
| Compared to baseline at the end of treatment‡ | -16.5(-19.9,-13.1) | -11.3(-15.4,-7.2) | 1.3(-3.6,6.2) |
| Change from baseline vs. placebo‡§ | -17.8(-23.8,-11.8) | -12.6(-19.1,-6.1) | |

| **Weight change (%)** | | | |
|---|---|---|---|
| Change rate at the end of treatment compared to baseline‡ | -17.8(-21.8,-13.9) | -12.8(-17.5,-8.0) | 0.7(-5.0,6.4) |
| Percentage Difference Between CFB | -18.6(-25.5,-11.6) | -13.5(-21.0,-6.0) | |
| and Placebo‡§ | | | |

| **BMI(kg/m²)** | | | |
|---|---|---|---|
| Baseline† | 32.6(2.6) | 35.3(5.1) | 34.0(4.3) |
| Compared to baseline at the end of treatment‡ | -5.7(-7.0,-4.4) | -4.4(-5.9,-2.9) | 0.4(-1.5,2.2) |
| Percentage Difference Between CFB and Placebo‡§ | -6.0(-8.3,-3.8) | -4.7(-7.1,-2.4) | |

| **Waist circumference (cm)** | | | |
|---|---|---|---|
| Baseline† | 104.6(11.2) | 110.5(14.6) | 104.7(10.5) |
| Compared to baseline at the end of treatment‡ | -12.6(-16.8,-8.4) | -10.7(-15.7,-5.7) | 2.0(-4.0,7.9) |
| Percentage Difference Between CFB and Placebo‡§ | -14.6(-21.9,-7.3) | -12.7(-20.5,-4.8) | |

| **Systolic blood pressure (mmHg)** | | | |
|---|---|---|---|
| Baseline† | 121.0(8.9) | 124.9(10.4) | 122.6(8.6) |
| Compared to baseline at the end of treatment‡ | -13.0(-20.1,-6.0) | -11.4(-19.8,-3.0) | -2.8(-12.6,7.1) |
| Percentage Difference Between CFB and Placebo‡§ | -10.3(-22.4,1.9) | 8.7(-21.6,4.2) | |

| **Diastolic blood pressure (mmHg)** | | | |
|---|---|---|---|
| Baseline† | 76.4(6.1) | 80.7(5.9) | 82.1(2.9) |
| Compared to baseline at the end of treatment‡ | -6.6(-11.6,-1.5) | -9.6(-15.4,-3.8) | 0.6(-6.4,7.6) |
| Percentage Difference Between CFB and Placebo‡§ | -7.2(-16.1,1.8) | -10.2(-19.1,-1.3) | |

| **Triglycerides (mmol/L)** | | | |
|---|---|---|---|
| Baseline† | 2.1(1.0) | 1.7(0.6) | 1.8(0.6) |
| Compared to baseline at the end of treatment‡ | -1.2(-1.6,-0.9) | -0.9(-1.4,-0.4) | 0.1(-0.4,0.6) |
| Percentage Difference Between CFB and Placebo‡§ | -1.3(-1.9,-0.7) | -1.0(-1.7,-0.3) | |

| **Total cholesterol (mmol/L)** | | | |
|---|---|---|---|
| Baseline† | 5.3(0.9) | 5.0(0.8) | 5.6(1.0) |
| Compared to baseline at the end of treatment‡ | -0.7(-1.1,-0.2) | -1.1(-1.7,-0.5) | -0.2(-0.8,0.5) |
| Percentage Difference Between CFB and Placebo‡§ | -0.5(-1.3,0.2) | -0.9(-1.8,-0.04) | |

| **Fasting blood glucose (mmol/L)** | | | |
|---|---|---|---|
| Baseline† | 5.5(0.4) | 5.5(0.5) | 5.7(0.4) |
| Compared to baseline at the end of treatment‡ | -1.0(-1.3,-0.8) | -0.7(-1.0,-0.4) | -0.2(-0.6,0.2) |
| Percentage Difference Between CFB and Placebo‡§ | -0.8(-1.3,-0.4) | -0.6(-1.1,-0.03) | |

| **Adverse events (AE)** | | | |
|---|---|---|---|
| Treatment of any related adverse events \|\| | 9(56.3) | 5(50) | 3(33.3) |
| Gastrointestinal adverse events \|\| | 9(56.3) | 4(40) | 3(33.3) |

| | | | |
|---|---|---|---|
| Note:*Efficacy was analyzed using the full analysis set (FAS), and safety was analyzed using the safety set (SS). Both the FAS and SS included all randomized subjects who received at least one dose of study drug. All changes were assessed from baseline to Week 35. **One subject in the compound (I) group discontinued the study after randomization and prior to dosing. †Data are expressed as the mean (SD). ‡Data are shown as least squares means with 95% confidence intervals. §Calculate the least squares mean difference from the ANCOVA model using the baseline value as a covariate. ∥Data are expressed as the number of subjects (percentage). ¶Gastrointestinal adverse events include nausea, vomiting, diarrhea, constipation, indigestion, and dysphagia. | | | |

AE, Adverse Event; BMI, Body Mass Index; CFB, Change from Baseline; EOT, End of Treatment; IP, Investigational Product; TRAE, Treatment-Related Adverse Event; QW, Once Weekly; Q2W, Once Every 2 Weeks.

Table 9 shows that at a frequency of once weekly, compound (I) resulted in an average weight reduction of 18.6% compared to placebo. At a frequency of once every two weeks, compound (I) resulted in an average weight reduction of 13.5% compared to placebo. There were no IP-related serious adverse events.

### Example 5: Clinical Dosage Regimen

A multicenter, randomized, double-blind, placebo-controlled Phase III clinical study in Chinese adult obese/overweight subjects was conducted to evaluate the efficacy and safety of compound (I) injection in adult obese/overweight subjects.

The trial enrolled obese/overweight adult subjects (≥18 years of age) who had failed to achieve adequate weight control through diet and exercise.

This study plans to enroll 630 adult obese/overweight subjects. Eligible subjects will be randomly assigned in a 1:1 ratio and stratified by baseline BMI to Group A (24 mg group, 315 subjects) and Group B (48 mg group, 315 subjects). Within Groups A and B, subjects will be further stratified and randomized in a 4:1 ratio to receive either compound (I) (n=252) or placebo (n=63), with double-blinding maintained within each group.

All subjects maintained regular dietary and exercise habits throughout the study period. Subject inclusion and exclusion criteria are described in Table 2.

Subjects were randomized to receive either compound (I) injection or an equal volume of placebo according to the dosage regimen in Table 10.

**Table 10: Dose-Escalation Dosage Regimen for Compound (I) Injection in Weight Loss Project**

| **Group** | **Target dose** | **Dosage Regimen** | | | **Number of subjects** | |
|---|---|---|---|---|---|---|
| | | **Sched ule (Week /W)** | **Dosage Week** | **Dose** | **Compound (I)** | **Placebo** |
| A | 24mg | 4W | WO/2 | 3mg | 252 | 63 |
| | | 4W | W4/6 | 6mg | | |
| | | 4W | W8/10 | 9mg | | |
| | | 4W | W12/14 | 12mg | | |
| | | 4W | W16/18 | 18mg | | |
| | | 28W | W20/22/24/26/28/ 30/32/34 /36/38/40/42/44/4 6 | 24mg | | |
| B | 48mg | 4W | WO/2 | 3mg | 252 | 63 |
| | | 4W | W4/6 | 6mg | | |
| | | 4W | W8/10 | 9mg | | |
| | | 4W | W12/14 | 12mg | | |
| | | 4W | W16/18 | 18mg | | |
| | | 4W | W20/22 | 24mg | | |
| | | 4W | W24/26 | 36mg | | |
| | | 20W | W28/30/32/34/36/ 38/40/42 /44/46 | 48mg | | |

The present invention has been described through the above examples, but it should be understood that the above examples are provided for illustrative purposes only and are not intended to limit the scope of the invention to the described examples. Furthermore, those skilled in the art will appreciate that the present invention is not limited to the above examples. Additional variations and modifications may be made based on the teachings of the present invention, and such variations and modifications fall within the scope of protection claimed herein. The scope of protection of the present invention is defined by the appended claims and their equivalents.

Sequences :
SEQ ID NO.1:
   GLP-1-(7-37) peptide 
His Ala Glu Gly Thr Phe Thr Ser Asp Val Ser Ser Tyr Leu Glu Gly Gln Ala Ala Lys Glu Phe Ile Ala
Trp Leu Val Lys Gly Arg Gly
SEQ ID NO.2 :
   [Gly8,Arg34]GLP-1-(7-37) peptide

His Gly Glu Gly Thr Phe Thr Ser Asp Val Ser Ser Tyr Leu Glu Gly Gln Ala Ala Lys Glu Phe Ile Ala
Trp Leu Val Arg Gly Arg Gly

## Claims

1. A method for treating or preventing obesity, wherein the method comprises administering to a subject in need thereof about 0.25 mg to about 100 mg of a compound of formula (I), or a pharmaceutically acceptable salt, amide, or ester thereof,

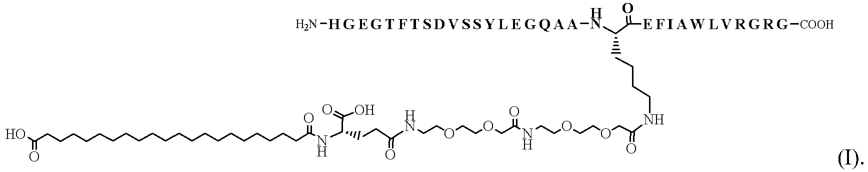

2. The method according to claim 1, wherein the method comprises administering to a subject in need thereof about 0.25-100 mg, preferably about 0.5-90 mg, preferably about 1-80 mg, preferably about 1.5-80 mg, preferably about 2-80 mg, preferably about 1.5-70 mg, preferably about 2-70 mg, preferably about 3-70 mg, preferably about 1.5-60 mg, preferably about 3-60 mg, preferably about 1.5-55 mg, preferably about 3-55 mg, preferably about 1.5-48 mg, preferably about 3-48 mg, preferably about 1.5-36 mg, preferably about 3-36 mg, preferably about 1.5-30 mg, preferably about 3-30 mg, preferably about 1.5-24 mg, preferably about 3-24 mg, preferably about 1.5-18 mg, preferably about 3-18 mg, preferably about 1.5-12 mg, preferably about 3-12 mg, preferably about 4-60 mg, preferably about 5-60 mg, preferably about 7-60 mg, preferably about 8-60 mg, preferably about 9-60 mg, preferably about 10-60 mg, preferably about 11-60 mg, preferably about 12-60 mg, preferably about 6-48 mg, preferably about 7-48 mg, preferably about 9-48 mg, preferably about 12-48 mg, preferably about 15-48 mg, or preferably about 24-48 mg of the compound of formula (I), or the pharmaceutically acceptable salt, amide, or ester thereof.

3. The method according to claim 1 or 2, wherein the method comprises administering to a subject in need thereof the compound of formula (I), or the pharmaceutically acceptable salt, amide, or ester thereof, at a weekly or biweekly dose of about 0.25-100 mg, preferably about 0.5-90 mg, preferably about 1-80 mg, preferably about 1.5-80 mg, preferably about 2-80 mg, preferably about 1.5-70 mg, preferably about 2-70 mg, preferably about 3-70 mg, preferably about 3-60 mg, preferably about 1.5-55 mg, preferably about 3-55 mg, preferably about 1.5-48 mg, preferably about 3-48 mg, preferably about 1.5-36 mg, preferably about 3-36 mg, preferably about 1.5-30 mg, preferably about 3-30 mg, preferably about 1.5-24 mg, preferably about 3-24 mg, preferably about 1.5-18 mg, preferably about 3-18 mg, preferably about 1.5-12 mg, preferably about 3-12 mg, preferably about 4-60 mg, preferably about 5-60 mg, preferably about 7-60 mg, preferably about 8-60 mg, preferably about 9-60 mg, preferably about 10-60 mg, preferably about 11-60 mg, preferably about 12-60 mg, preferably about 6-48 mg, preferably about 7-48 mg, preferably about 9-48 mg, preferably about 12-48 mg, preferably about 15-48 mg, preferably about 24-48 mg.

4. The method according to any one of claims 1 to 3, wherein the method comprises administering to a subject in need thereof the compound of formula (I), or the pharmaceutically acceptable salt, amide, or ester thereof, at a weekly or biweekly dose of about 0.25 mg, about 0.5 mg, about 1 mg, about 1.5 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, or about 70 mg.

5. The method according to any one of claims 1 to 4, wherein the method comprises administering to a subject in need thereof the compound of formula (I), or the pharmaceutically acceptable salt, amide, or ester thereof, once weekly or less frequently, preferably once every two weeks or less frequently.

6. The method according to any one of claims 1 to 5, wherein the method comprises administering to a subject in need thereof the compound of formula (I), or the pharmaceutically acceptable salt, amide, or ester thereof, at a frequency of once weekly or once every two weeks, at the following dose: about 0.25 mg, about 0.5 mg, about 1 mg, about 1.5 mg, about 2 mg, about 3 mg, about 4 mg, about 5 mg, about 6 mg, about 7 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, or about 70 mg.

7. The method according to any one of claims 1 to 6, wherein the method comprises first administering an escalation dose to a subject in need thereof at a frequency of once weekly or once every two weeks during an escalation dose period, and subsequently administering a maintenance dose at a frequency of once weekly or once every two weeks during a maintenance dose period,
wherein the escalation dose period is at least two weeks, preferably about 2 to 60 weeks, more preferably about 2 to 55 weeks, more preferably about 2 to 50 weeks, more preferably about 2 to 40 weeks, more preferably about 2 to 31 weeks, more preferably about 2 to 28 weeks, more preferably about 2 to 30 weeks, more preferably about 2 to 20 weeks, and preferably the escalation dose period is about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, about 52 weeks, about 53 weeks, about 54 weeks, about 55 weeks, about 56 weeks, about 57 weeks, about 58 weeks, about 59 weeks, or about 60 weeks;
the maintenance dose period is at least about two weeks, preferably about 3 weeks or more, about 4 weeks or more, about 5 weeks or more, about 6 weeks or more, about 7 weeks or more, about 8 weeks or more, about 9 weeks or more, about 10 weeks or more, about 11 weeks or more, about 12 weeks or more or more, about 13 weeks, about 14 weeks or more, about 15 weeks or more, about 16 weeks or more, about 17 weeks or more, about 18 weeks or more, about 19 weeks or more, about 20 weeks or more, about 21 weeks or more, about 22 weeks or more, about 23 weeks or more, about 24 weeks or more, about 25 weeks or more, about 26 weeks or more, about 27 weeks or more, about 28 weeks or more, about 29 weeks or more, about 30 weeks or more, about 31 weeks or more, about 32 weeks or more, about 33 weeks or more, about 34 weeks or more, about 35 weeks or more, about 36 weeks or more, about 37 weeks or more, about 38 weeks or more, about 39 weeks or more, about 40 weeks or more, about 41 weeks or more, about 42 weeks or more, about 43 weeks or more, about 44 weeks or more, about 45 weeks or more, about 46 weeks or more, about 47 weeks or more, about 48 weeks or more, about 49 weeks or more, about 50 weeks or more, about 51 weeks or more, about 52 weeks or more, about 53 weeks or more, about 54 weeks or more, about 55 weeks or more, about 56 weeks or more, about 57 weeks or more, about 58 weeks or more, about 59 weeks or more, or about 60 weeks or more; preferably about 2-120 weeks, preferably about 2-100 weeks, preferably about 2-80 weeks, preferably about 2-70 weeks, preferably about 2-60 weeks, preferably about 2-55 weeks, preferably about 2-50 weeks, preferably about 2-40 weeks, preferably about 2-35 weeks, preferably about 2-30 weeks, preferably about 2-20 weeks; preferably the maintenance dosage period is about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, about 30 weeks, about 31 weeks, about 32 weeks, about 33 weeks, about 34 weeks, about 35 weeks, about 36 weeks, about 37 weeks, about 38 weeks, about 39 weeks, about 40 weeks, about 41 weeks, about 42 weeks, about 43 weeks, about 44 weeks, about 45 weeks, about 46 weeks, about 47 weeks, about 48 weeks, about 49 weeks, about 50 weeks, about 51 weeks, about 52 weeks, about 53 weeks, about 54 weeks, about 55 weeks, about 56 weeks, about 57 weeks, about 58 weeks, about 59 weeks, or about 60 weeks;
the escalation dose is independently about 0.25mg-70mg of the compound of formula (I), or the pharmaceutically acceptable salt, amide or ester thereof per administration; preferably, the escalation dose is independently about 0.25 mg, about 0.5 mg, about 1 mg, about 1.5 mg, about 2 mg, about 3 mg, about 4 mg, about 5 mg, about 6 mg, about 7 mg, about 7.5 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, or about 70 mg of the compound of formula (I), or the pharmaceutically acceptable salt, amide, or ester thereof per administration;
the maintenance dose is about 3-70 mg of the compound of formula (I), or the pharmaceutically acceptable salt, amide, or ester thereof; preferably, the maintenance dosage is about 3 mg, about 4 mg, about 5 mg, about 6 mg, about 7 mg, about 7.5 mg, about 8 mg, about 9 mg, about 10 mg, about 11 mg, about 12 mg, about 13 mg, about 14 mg, about 15 mg, about 16 mg, about 17 mg, about 18 mg, about 19 mg, about 20 mg, about 21 mg, about 22 mg, about 23 mg, about 24 mg, about 25 mg, about 26 mg, about 27 mg, about 28 mg, about 29 mg, about 30 mg, about 31 mg, about 32 mg, about 33 mg, about 34 mg, about 35 mg, about 36 mg, about 37 mg, about 38 mg, about 39 mg, about 40 mg, about 41 mg, about 42 mg, about 43 mg, about 44 mg, about 45 mg, about 46 mg, about 47 mg, about 48 mg, about 49 mg, about 50 mg, about 51 mg, about 52 mg, about 53 mg, about 54 mg, about 55 mg, about 56 mg, about 57 mg, about 58 mg, about 59 mg, about 60 mg, about 61 mg, about 62 mg, about 63 mg, about 64 mg, about 65 mg, about 66 mg, about 67 mg, about 68 mg, about 69 mg, or about 70 mg of the compound of formula (I), or the pharmaceutically acceptable salt, amide, or ester thereof; and
the maintenance dose is greater than or equal to the escalation dose;
preferably:
during the escalation dose period, the administration period for each different escalation dose is independently about 1 week or more, about 2 weeks or more, about 3 weeks or more, about 4 weeks or more, about 5 weeks or more, about 6 weeks or more, about 7 weeks or more, about 8 weeks or more, about 9 weeks or more, about 10 weeks or more, about 11 weeks, about 12 weeks or more, about 13 weeks or more, about 14 weeks or more, about 15 weeks or more, about 16 weeks or more, about 17 weeks or more, about 18 weeks, about 19 weeks or more, or about 20 weeks or more; preferably, during the escalation dose period, the administration period for each different escalation dose is independently about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, or about 20 weeks;
preferably:
during the escalation dose period, the escalation dose is about 1.1-3 times the preceding different escalation dose, preferably about 1.1-2 times, more preferably about 1.1 times, about 1.2 times, about 1.3 times, about 1.4 times, about 1.5 times, about 1.6 times, about 1.7 times, about 1.8 times, about 1.9 times, about 2.1 times, about 2.2 times, about 2.3 times, about 2.4 times, about 2.5 times, about 2.6 times, about 2.7 times, about 2.8 times, about 2.9 times, or about 3 times.

8. The method according to claim 7, wherein the escalation dose period is about 8-31 weeks, preferably about 10-31 weeks, preferably the escalation dose period is about 10 weeks, about 12 weeks, about 14 weeks, about 16 weeks, about 18 weeks, about 20 weeks, about 26 weeks, about 27 weeks, about 28 weeks, about 30 weeks or about 31 weeks; preferably the escalation dose period is about 10 weeks, about 14 weeks, about 18 weeks, about 28 weeks, or about 31 weeks;
the maintenance dose period is about 4 weeks or more, 5 weeks or more, about 12 weeks or more, about 14 weeks or more, about 16 weeks or more, about 18 weeks or more, about 20 weeks or more, about 30 weeks or more, about 40 weeks or more, about 50 weeks or more, about 60 weeks or more, about 70 weeks or more, about 80 weeks or more, or about 90 weeks or more; preferably the maintenance dose period is about 4-20 weeks, preferably the maintenance dose period is about 4 weeks, about 5 weeks, about 12 weeks, about 14 weeks, about 16 weeks, about 18 weeks, or about 20 weeks;
the escalation dose is independently about 1.5mg, about 3mg, about 5mg, about 6mg, about 7mg, about 7.5mg, about 9mg, about 12mg, about 15mg, about 18mg, about 24mg, about 30mg, about 36mg, or about 48mg of the compound of formula (I), or the pharmaceutically acceptable salt, amide, or ester thereof per administration; preferably, the escalation dose is independently about 3mg, about 6mg, about 9mg, about 12mg, about 15mg, about 18mg, about 24mg, or about 36mg of the compound of formula (I), or the pharmaceutically acceptable salt, amide, or ester thereof per administration; or the escalation dose is independently about 1.5mg, about 3mg, about 5mg, about 7mg, about 9mg, about 12mg, about 15mg, about 18mg, or about 24mg of the compound of formula (I), or the pharmaceutically acceptable salt, amide, or ester thereof per administration; or the escalation dose is independently about 3mg, about 6mg, about 9mg, about 12mg, about 18mg, about 24mg, about 36mg or 48mg of the compound of formula (I), or the pharmaceutically acceptable salt, amide or ester thereof per administration;
the maintenance dose is about 5mg, about 12mg, about 18mg, about 24mg, about 30mg, about 36mg, or about 48mg of the compound of formula (I), or the pharmaceutically acceptable salt, amide, or ester thereof; and
the maintenance dose is greater than or equal to the escalation dose.

9. The method according to claim 7 or 8, wherein the method comprises first administering the escalation dose to a subject in need thereof at a frequency of once weekly or once every two weeks during the escalation dose period, and then administering the maintenance dose at a frequency of once weekly or once every two weeks during the maintenance dose period, wherein,
the escalation dose period is about 10 weeks, about 14 weeks, about 18 weeks, about 28 weeks, about 30 weeks or about 31 weeks;
the maintenance dose period is about 4 weeks or more, about 12 weeks or more, about 16 weeks or more, or about 20 weeks or more; preferably, the maintenance dose period is about 4 weeks, about 12 weeks, about 16 weeks, or about 20 weeks;
the escalation dose is independently about 1.5mg, about 3mg, about 5mg, about 6mg, about 7mg, about 9mg, about 12mg, about 15mg, about 18mg, about 24mg, about 30mg, about 36mg or about 48mg of the compound of formula (I), or the pharmaceutically acceptable salt, amide or ester thereof per administration;
the maintenance dose is about 5mg, about 12mg, about 18mg, about 24mg, about 30mg, about 36mg, or about 48mg of the compound of formula (I), or the pharmaceutically acceptable salt, amide, or ester thereof; and
the maintenance dose is greater than or equal to the escalation dose;
preferably:
when the maintenance dose is about 12mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide, or ester thereof at a frequency of once every two weeks for about 2 or more or 4 weeks or more; administering about 6mg of the compound of formula (I), or the pharmaceutically acceptable salt, amide or ester thereof at a frequency of once every two weeks for about 4 weeks or more; administering about 9mg of the compound of formula (I), or the pharmaceutically acceptable salt, amide or ester thereof at a frequency of once every two weeks for about 4 weeks or more; and administering about 12mg of the compound of formula (I), or the pharmaceutically acceptable salt, amide or ester thereof at a frequency of once every two weeks for 20 weeks or more; preferably for 25 weeks or more, preferably for 30 weeks or more, preferably for about 20-120 weeks, preferably for about 20-100 weeks, preferably for about 20-80 weeks; preferably for about 20-60 weeks; or
when the maintenance dose is about 18mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once every two weeks for about 2 weeks or more or 4 weeks or more; administering about 6mg of the compound of formula (I), or the pharmaceutically acceptable salt, amide or ester thereof at a frequency of once every two weeks for about 4 weeks or more; administering about 9mg of the compound of formula (I), or the pharmaceutically acceptable salt, amide or ester thereof at a frequency of once every two weeks for about 4 weeks or more, administering about 12mg of the compound of formula (I), or the pharmaceutically acceptable salt, amide or ester thereof at a frequency of once every two weeks for about 4 weeks or more; and administering about 18mg of the compound of formula (I), or the pharmaceutically acceptable salt, amide or ester thereof at a frequency of once every two weeks for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
when the maintenance dose is about 24mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once every two weeks for about 2 weeks or more or 4 weeks or more; administering about 6mg of the compound of formula (I), or the pharmaceutically acceptable salt, amide or ester thereof at a frequency of once every two weeks for about 4 weeks or more; administering about 9mg of the compound of formula (I), or the pharmaceutically acceptable salt, amide or ester thereof at a frequency of once every two weeks for about 4 weeks or more administering about 12mg of the compound of formula (I), or the pharmaceutically acceptable salt, amide or ester thereof at a frequency of once every two weeks for about 4 weeks or more; administering about 18mg of the compound of formula (I), or the pharmaceutically acceptable salt, amide or ester thereof at a frequency of once every two weeks for about 4 weeks or more; administering about 24mg of the compound of formula (I), or the pharmaceutically acceptable salt, amide or ester thereof at a frequency of once every two weeks for 12 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
when the maintenance dose is about 48mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once every two weeks for about 2 or more or 4 weeks or more; administering about 6mg of the compound of formula (I), or the pharmaceutically acceptable salt, amide or ester thereof at a frequency of once every two weeks for about 4 weeks or more; administering about 12mg of the compound of formula (I), or the pharmaceutically acceptable salt, amide or ester thereof at a frequency of once every two weeks for about 4 weeks or more; administering about 24mg of the compound of formula (I), or the pharmaceutically acceptable salt, amide or ester thereof at a frequency of once every two weeks for about 4 weeks or more; administering about 36mg of the compound of formula (I), or the pharmaceutically acceptable salt, amide or ester thereof at a frequency of once every two weeks for about 4 weeks or more; administering about 48mg of the compound of formula (I), or the pharmaceutically acceptable salt, amide or ester thereof at a frequency of once every two weeks for 12 weeks or more, preferably for 14 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
when the maintenance dose is about 48mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once every two weeks for about 2 weeks or more or 4 weeks or more; administering about 6mg of the compound of formula (I), or the pharmaceutically acceptable salt, amide or ester thereof at a frequency of once every two weeks for about 4 weeks or more; administering about 9mg of the compound of formula (I), or the pharmaceutically acceptable salt, amide or ester thereof at a frequency of once every two weeks for about 4 weeks or more; administering about 12mg of the compound of formula (I), or the pharmaceutically acceptable salt, amide or ester thereof at a frequency of once every two weeks for about 4 weeks or more; administering about 18mg of the compound of formula (I), or the pharmaceutically acceptable salt, amide or ester thereof at a frequency of once every two weeks for about 4 weeks or more; administering about 24mg of the compound of formula (I), or the pharmaceutically acceptable salt, amide or ester thereof at a frequency of once every two weeks for about 4 weeks or more; administering about 36mg of the compound of formula (I), or the pharmaceutically acceptable salt, amide or ester thereof at a frequency of once every two weeks for about 4 weeks or more; administering about 48mg of the compound of formula (I), or the pharmaceutically acceptable salt, amide or ester thereof at a frequency of once every two weeks for 12 weeks or more, preferably for 14 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
when the maintenance dose is about 36mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once every two weeks for about 2 weeks or more or 4 weeks or more; administering about 6mg of the compound of formula (I), or the pharmaceutically acceptable salt, amide or ester thereof at a frequency of once every two weeks for about 4 weeks or more; administering about 12mg of the compound of formula (I), or the pharmaceutically acceptable salt, amide or ester thereof at a frequency of once every two weeks for about 4 weeks or more; administering about 18mg of the compound of formula (I), or the pharmaceutically acceptable salt, amide or ester thereof at a frequency of once every two weeks for about 4 weeks or more; administering about 24mg of the compound of formula (I), or the pharmaceutically acceptable salt, amide or ester thereof at a frequency of once every two weeks for about 4 weeks or more; and administering about 36mg of the compound of formula (I), or the pharmaceutically acceptable salt, amide or ester thereof at a frequency of once every two weeks for 12 weeks or more, preferably for 14 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
when the maintenance dose is about 24mg of the compound of formula (I), or the pharmaceutically acceptable salt, amide or ester thereof, the method comprises sequentially administering to a subject in need thereof about 3mg of the compound of formula (I), or the pharmaceutically acceptable salt, amide or ester thereof at a frequency of once weekly for about 2 weeks or more or 4 weeks or more; administering about 6mg of the compound of formula (I), or the pharmaceutically acceptable salt, amide or ester thereof once weekly for 4 weeks or more; administering about 9mg of the compound of formula (I), or the pharmaceutically acceptable salt, amide or ester thereof at a frequency of once weekly for 4 weeks or more; administering about 12mg of the compound of formula (I), or the pharmaceutically acceptable salt, amide or ester thereof at a frequency of once weekly for 4 weeks or more; administering about 15mg of the compound of formula (I), or the pharmaceutically acceptable salt, amide or ester thereof at a frequency of once weekly for 4 weeks or more; and administering about 24mg of the compound of formula (I), or the pharmaceutically acceptable salt, amide or ester thereof at a frequency of once weekly for 12 weeks or more, preferably for 14 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks; or
when the maintenance dose is about 30mg of the compound of formula (I), or the pharmaceutically acceptable salt, amide or ester thereof, the method comprises sequentially administering to a subject in need thereof about 1.5mg of the compound of formula (I), or the pharmaceutically acceptable salt, amide or ester thereof at a frequency of once weekly for about 1 week or more; administering about 3mg of the compound of formula (I), or the pharmaceutically acceptable salt, amide or ester thereof at a frequency of once weekly for 2 weeks or more; administering about 5mg of the compound of formula (I), or the pharmaceutically acceptable salt, amide or ester thereof at a frequency of once weekly for 4 weeks or more; administering about 7mg of the compound of formula (I), or the pharmaceutically acceptable salt, amide or ester thereof at a frequency of once weekly for 4 weeks or more; administering about 9mg of the compound of formula (I), or the pharmaceutically acceptable salt, amide or ester thereof at a frequency of once weekly or once every two weeks for 4 weeks or more; administering about 12mg of the compound of formula (I), or the pharmaceutically acceptable salt, amide or ester thereof at a frequency of once weekly or once every two weeks for 4 weeks or more; administering about 15mg of the compound of formula (I), or the pharmaceutically acceptable salt, amide or ester thereof at a frequency of once weekly or once every two weeks for 4 weeks or more; administering about 18mg of the compound of formula (I), or the pharmaceutically acceptable salt, amide or ester thereof at a frequency of once weekly or once every two weeks for 4 weeks or more; administering about 24mg of the compound of formula (I), or the pharmaceutically acceptable salt, amide or ester thereof at a frequency of once weekly or once every two weeks for about 4 weeks or more; and administering about 30mg of a compound of formula (I), or the pharmaceutically acceptable salt, amide or ester thereof at a frequency of once weekly or once every two weeks for 4 weeks or more, preferably for 14 weeks or more, preferably for 16 weeks or more, preferably for 18 weeks or more, preferably for 20 weeks or more, preferably for 25 weeks or more, preferably for 30 weeks or more, preferably for about 20-120 weeks, preferably for about 20-100 weeks, preferably for about 20-80 weeks; preferably for about 20-60 weeks.

10. The method according to any one of claims 1-6 or 7-9, wherein the method comprises administering to a subject in need thereof a first dose of about 0.25-5mg of the compound of formula (I), or the pharmaceutically acceptable salt, amide or ester thereof at a frequency of once weekly or once every two weeks;
after administering the first dose for at least about 1 week, preferably about 1, about 2, about 3, about 4, about 5, about 6, about 7, or about 8 weeks, administering a second dose of about 3-12mg of the compound of formula (I), or the pharmaceutically acceptable salt, amide, or ester thereof at a frequency of once weekly or once every two weeks; preferably, after administering the first dose for about 1, about 2, about 3 or about 4 weeks, administering the second dose of about 3-12mg of the compound of formula (I), or the pharmaceutically acceptable salt, amide or ester thereof at a frequency of once weekly or once every two weeks;
after administering the second dose for about 2, about 3, about 4, about 5, about 6, about 7, or about 8 weeks, administering a third dose of about 6-24mg of the compound of formula (I), or the pharmaceutically acceptable salt, amide, or ester thereof at a frequency of once weekly or once every two weeks; and
after administering the third dose for about 3, about 4, about 5, about 6, about 7, or about 8 weeks, administering a fourth dose of about 9-36mg of the compound of formula (I), or the pharmaceutically acceptable salt, amide, or ester thereof at a frequency of once weekly or once every two weeks.

11. The method according to claim 10, wherein after administering the fourth dose for about 3, about 4, about 5, about 6, about 7, about 8, about 9, about 10, about 11, about 12, about 13, about 14, about 15, about 16, about 17, about 18, about 19, or about 20 weeks, administering a fifth dose of about 12-48mg of the compound of formula (I) or the pharmaceutically acceptable salt, amide, or ester thereof at a frequency of once weekly or once every two weeks.

12. The method according to claim 11, wherein after administering the fifth dose for about 3, about 4, about 5, about 6, about 7, about 8, about 9, about 10, about 11, about 12, about 13, about 14, about 15, about 16, about 17, about 18, about 19, or about 20 weeks, administering a sixth dose of about 18-60mg of the compound of formula (I) or the pharmaceutically acceptable salt, amide, or ester thereof at a frequency of once weekly or once every two weeks.

13. The method according to claim 12, wherein after administering the sixth dose for about 2, about 3, about 4, about 5, about 6, about 7, about 8, about 9, about 10, about 11, about 12, about 13, about 14, about 15, about 16, about 17, about 18, about 19, or about 20 weeks, administering a seventh dose of about 24-72mg of the compound of formula (I), or the pharmaceutically acceptable salt, amide, or ester thereof at a frequency of once weekly or once every two weeks; optionally, after further administering the seventh dose for about 2, about 3, about 4, about 5, about 6, about 7, about 8, about 9, about 10, about 11, about 12, about 13, about 14, about 15, about 16, about 17, about 18, about 19, or about 20 weeks, administering an eighth dose of about 30-84mg of the compound of formula (I), or the pharmaceutically acceptable salt, amide, or ester thereof at a frequency of once weekly or once every two weeks.

14. The method according to any one of claims 10-13, wherein the first dose is about 0.5-5mg, preferably about 1-4.5mg, preferably about 1.5-4mg, preferably about 1.5-3.5mg, preferably about 2-3mg, preferably about 3mg; and/or
the second dose is about 3-7.5mg, preferably about 3.5-7.5mg, preferably about 4-7mg, preferably about 4.5-6.5mg, preferably about 5-6mg, preferably about 6mg; and/or
the third dose is about 5-24mg, preferably about 6-20mg mg, preferably about 6-18mg, preferably about 6-12mg, preferably about 6mg, about 7mg, about 8mg, about 9mg, about 10mg, about 11mg, about 12mg, about 13mg, about 14mg, about 15mg, about 16mg, about 17mg, about 18mg, about 19mg, or about 20mg, preferably about 9mg or about 12mg; and/or
the fourth dose is about 7-40mg, preferably about 9-36mg, preferably about 9mg, about 10mg, about 11mg, about 12mg, about 13mg, about 14mg, about 15mg, about 16mg, about 17mg, about 18mg, about 19mg, about 20mg, about 21mg, about 22mg, about 23mg, about 24mg, about 25mg, about 26mg, about 27mg, about 28mg, about 29mg, about 30mg, about 31mg, about 32mg, about 33mg, about 34mg, about 35mg, or about 36mg, preferably about 12mg or about 24mg; and/or
the fifth dose is about 9-50mg, preferably about 12-48mg, preferably about 12mg, about 13mg, about 14mg, about 15mg, about 16mg, about 17mg, about 18mg, about 19mg, about 20mg, about 21mg, about 22mg, about 23mg, about 24mg, about 25mg, about 26mg, about 27mg, about 28mg, about 29mg, about 30mg, about 31mg, about 32mg, about 33mg, about 34mg, about 35mg, about 36mg, about 37mg, about 38mg, about 39mg, about 40mg, about 41mg, about 42mg, about 43mg, about 44mg, about 45mg, about 46mg, about 47mg or about 48mg, preferably about 15mg, about 18mg or about 36mg; and/or
the sixth dose is about 12-50mg, preferably about 18-60mg, preferably about 24mg, about 25mg, about 26mg, about 27mg, about 28mg, about 29mg, about 30mg, about 31mg, about 32mg, about 33mg, about 34mg, about 35mg, about 36mg, about 37mg, about 38mg, about 39mg, about 40mg, about 41mg, about 42mg, about 43mg, about 44mg, about 45mg, about 46mg, about 47mg, about 48mg, about 49mg, about 50mg, about 51mg, about 52mg, about 53mg, about 54mg, about 55mg, about 56mg, about 57mg, about 58mg, about 59mg, or about 60mg, preferably about 24mg or about 48mg; and/or
the seventh dose is about 15-75mg, preferably about 24-72mg, preferably about 24mg, about 25mg, about 26mg, about 27mg, about 28mg, about 29mg, about 30mg, about 31mg, about 32mg, about 33mg, about 34mg, about 35mg, about 36mg, about 37mg, about 38mg, about 39mg, about 40mg, about 41mg, about 42mg, about 43mg, about 44mg, about 45mg, about 46mg, about 47mg, about 48mg, about 49mg, about 50mg, about 51mg, about 52mg, about 53mg, about 54mg, about 55mg, about 56mg, about 57mg, about 58mg, about 59mg, or about 60mg, preferably about 30mg, about 36mg, or about 48mg; and/or
the eighth dose is about 30-84mg, preferably about 36-72mg, preferably about 30mg, about 31mg, about 32mg, about 33mg, about 34mg, about 35mg, about 36mg, about 37mg, about 38mg, about 39mg, about 40mg, about 41mg, about 42mg, about 43mg, about 44mg, about 45mg, about 46mg, about 47mg, about 48mg, about 49mg, about 50mg, about 51mg, about 52mg, about 53mg, about 54mg, about 55mg, about 56mg, about 57mg, about 58mg, about 59mg, about 60mg, about 61mg, about 62mg, about 63mg, about 64mg, about 65mg, about 66mg, about 67mg, about 68mg, about 69mg, about 70mg, about 71mg or about 72mg, preferably about 48mg.

15. The method according to any one of claims 1-6, 7-9 or 10-14, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 weeks or about 4 weeks, administering a second dose of about 6mg of the compound of formula (I), or the pharmaceutically acceptable salt, amide or ester thereof at a frequency of once weekly or once every two weeks;
after administering the second dose for about 4 weeks, administering a third dose of about 9mg of the compound of formula (I), or the pharmaceutically acceptable salt, amide, or ester thereof at a frequency of once weekly or once every two weeks; and
after administering the third dose for about 4 weeks, administering a fourth dose of about 12mg of the compound of formula (I), or the pharmaceutically acceptable salt, amide or ester thereof at a frequency of once weekly or once every two weeks, preferably administering the fourth dose of about 12mg of the compound of formula (I), or the pharmaceutically acceptable salt, amide or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably, administering the fourth dose of about 12mg of the compound of formula (I), or the pharmaceutically acceptable salt, amide, or ester thereof at the frequency of once weekly or once every two weeks for about 20 weeks.

16. The method according to any one of claims 1-6, 7-9 or 10-14, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 or 4 weeks, administering a second dose of about 6mg of the compound of formula (I), or the pharmaceutically acceptable salt, amide or ester thereof at a frequency of once weekly or once every two weeks;
after administering the second dose for about 4 weeks, administering a third dose of about 9mg of the compound of formula (I), or the pharmaceutically acceptable salt, amide, or ester thereof at a frequency of once weekly or once every two weeks;
after administering the third dose for about 4 weeks, administering a fourth dose of about 12mg of the compound of formula (I), or the pharmaceutically acceptable salt, amide, or ester thereof at a frequency of once weekly or once every two weeks; and
after administering the fourth dose for about 4 weeks, administering a fifth dose of about 18mg of the compound of formula (I), or the pharmaceutically acceptable salt, amide, or ester thereof at a frequency of once weekly or once every two weeks, preferably, administering the fifth dose of about 18mg of the compound of formula (I), or the pharmaceutically acceptable salt, amide, or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably, administering the fifth dose of about 18mg of the compound of formula (I), or the pharmaceutically acceptable salt, amide, or ester thereof at the frequency of once weekly or once every two weeks for about 16 weeks.

17. The method according to any one of claims 1-6, 7-9 or 10-14, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 or 4 weeks, administering a second dose of about 6mg of the compound of formula (I), or the pharmaceutically acceptable salt, amide, or ester thereof at a frequency of once weekly or once every two weeks;
after administering the second dose for about 4 weeks, administering a third dose of about 9mg of the compound of formula (I), or the pharmaceutically acceptable salt, amide, or ester thereof at a frequency of once weekly or once every two weeks;
after administering the third dose for about 4 weeks, administering a fourth dose of about 12mg of the compound of formula (I), or the pharmaceutically acceptable salt, amide, or ester thereof at a frequency of once weekly or once every two weeks;
after administering the fourth dose for about 4 weeks, administering a fifth dose of about 18mg of the compound of formula (I), or the pharmaceutically acceptable salt, amide, or ester thereof at a frequency of once weekly or once every two weeks; and
after administering the fifth dose for about 4 weeks, administering a sixth dose of about 24mg of the compound of formula (I), or the pharmaceutically acceptable salt, amide, or ester thereof at a frequency of once weekly or once every two weeks, preferably, administering the sixth dose of about 24mg of the compound of formula (I), or the pharmaceutically acceptable salt, amide, or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably, administering the sixth dose of about 24mg of the compound of formula (I), or the pharmaceutically acceptable salt, amide, or ester thereof at the frequency of once weekly or once every two weeks for about 12 weeks.

18. The method according to claims 1-6, 7-9 or 10-14, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 or 4 weeks, administering a second dose of about 6mg of the compound of formula (I), or the pharmaceutically acceptable salt, amide, or ester thereof at a frequency of once weekly or once every two weeks;
after administering the second dose for about 4 weeks, administering a third dose of about 9mg of the compound of formula (I), or the pharmaceutically acceptable salt, amide, or ester thereof at a frequency of once weekly or once every two weeks;
after administering the third dose for about 4 weeks, administering a fourth dose of about 12mg of the compound of formula (I), or the pharmaceutically acceptable salt, amide, or ester thereof at a frequency of once weekly or once every two weeks;
after administering the fourth dose for about 4 weeks, administering a fifth dose of about 18mg of the compound of formula (I), or the pharmaceutically acceptable salt, amide, or ester thereof once weekly or once every two weeks;
after administering the fifth dose for about 4 weeks, administering a sixth dose of about 24mg of the compound of formula (I), or the pharmaceutically acceptable salt, amide, or ester thereof once weekly or once every two weeks; and
after administering the sixth dose for about 4 weeks, administering a seventh dose of about 48mg of the compound of formula (I), or the pharmaceutically acceptable salt, amide or ester thereof at a frequency of once weekly or once every two weeks, preferably administering the seventh dose of about 48mg of the compound of formula (I), or the pharmaceutically acceptable salt, amide or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more;
preferably:
the method comprises administering to a subject in need thereof the first dose of about 3mg of the compound of formula (I), or the pharmaceutically acceptable salt, amide or ester thereof at the frequency of once weekly or once every two weeks;
after administering the first dose for about 2 or 4 weeks, administering the second dose of about 6mg of the compound of formula (I), or the pharmaceutically acceptable salt, amide, or ester thereof at the frequency of once weekly or once every two weeks;
after administering the second dose for about 4 weeks, administering the third dose of about 9mg of the compound of formula (I), or the pharmaceutically acceptable salt, amide, or ester thereof at the frequency of once weekly or once every two weeks;
after administering the third dose for about 4 weeks, administering the fourth dose of about 12mg of the compound of formula (I), or the pharmaceutically acceptable salt, amide, or ester thereof at the frequency of once weekly or once every two weeks;
after administering the fourth dose for about 4 weeks, administering the fifth dose of about 18mg of the compound of formula (I), or the pharmaceutically acceptable salt, amide, or ester thereof at the frequency of once weekly or once every two weeks;
after administering the fifth dose for about 4 weeks, administering the sixth dose of about 24mg of the compound of formula (I), or the pharmaceutically acceptable salt, amide, or ester thereof at the frequency of once weekly or once every two weeks; and
after administering the sixth dose for about 4 weeks, administering the seventh dose of about 36mg of the compound of formula (I), or the pharmaceutically acceptable salt, amide, or ester thereof at the frequency of once weekly or once every two weeks;
after administering the seventh dose for about 4 weeks, administering the eighth dose of about 48mg of the compound of formula (I), or the pharmaceutically acceptable salt, amide or ester thereof at the frequency of once weekly or once every two weeks, preferably administering the eighth dose of about 48mg of the compound of formula (I), or the pharmaceutically acceptable salt, amide or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more.

19. The method according to claims 1-6, 7-9 or 10-14, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 or 4 weeks, administering a second dose of about 6mg of the compound of formula (I), or the pharmaceutically acceptable salt, amide, or ester thereof at a frequency of once weekly or once every two weeks;
after administering the second dose for about 4 weeks, administering a third dose of about 12mg of the compound of formula (I), or the pharmaceutically acceptable salt, amide or ester thereof at a frequency of once weekly or once every two weeks;
after administering the third dose for about 4 weeks, administering a fourth dose of about 24mg of the compound of formula (I), or the pharmaceutically acceptable salt, amide or ester thereof at a frequency of once weekly or once every two weeks;
after administering the fourth dose for about 4 weeks, administering a fifth dose of about 36mg of the compound of formula (I), or the pharmaceutically acceptable salt, amide or ester thereof at a frequency of once weekly or once every two weeks; and
after administering the fifth dose for about 4 weeks, administering a sixth dose of about 48mg of the compound of formula (I) or the pharmaceutically acceptable salt, amide or ester thereof at a frequency of once weekly or once every two weeks, preferably administering the sixth dose of about 48mg of the compound of formula (I), or the pharmaceutically acceptable salt, amide or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably administering the sixth dose of about 48mg of the compound of formula (I), or the pharmaceutically acceptable salt, amide or ester thereof at the frequency of once weekly or once every two weeks for 12 weeks or more.

20. The method according to claims 1-6, 7-9 or 10-14, wherein the method comprises administering to a subject in need thereof a first dose of about 3mg of the compound of formula (I), or the pharmaceutically acceptable salt, amide or ester thereof at a frequency of once weekly or once every two weeks;
after administering the first dose for about 2 or 4 weeks, administering a second dose of about 6mg of the compound of formula (I), or the pharmaceutically acceptable salt, amide, or ester thereof at a frequency of once weekly or once every two weeks;
after administering the second dose for about 4 weeks, administering a third dose of about 9mg of the compound of formula (I), or the pharmaceutically acceptable salt, amide, or ester thereof at a frequency of once weekly or once every two weeks;
after administering the third dose for about 4 weeks, administering a fourth dose of about 12mg of the compound of formula (I), or the pharmaceutically acceptable salt, amide, or ester thereof at a frequency of once weekly or once every two weeks;
after administering the fourth dose for 4 weeks, administering a fifth dose of about 15mg of the compound of formula (I), or the pharmaceutically acceptable salt, amide or ester thereof at a frequency of once weekly or once every two weeks; and
after administering the fifth dose for about 4 weeks, administering a sixth dose of about 24mg of the compound of formula (I), or the pharmaceutically acceptable salt, amide, or ester thereof at a frequency of once weekly or once every two weeks, preferably administering the six dose of about 24mg of the compound of formula (I), or the pharmaceutically acceptable salt, amide, or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably, administering the sixth dose of about 24mg of the compound of formula (I), or the pharmaceutically acceptable salt, amide or ester thereof at the frequency of once weekly or once every two weeks for about 12 weeks or more.

21. The method according to any one of claims 1-6 or 7-9, wherein the method comprises administering to a subject in need thereof a first' dose of about 0.25-3mg of the compound of formula (I), or the pharmaceutically acceptable salt, amide or ester thereof at a frequency of once weekly or once every two weeks; and/or
after administering the first' dose for about 1, about 2, about 3, or about 4 weeks, administering a second' dose of about 1-5mg of the compound of formula (I), or the pharmaceutically acceptable salt, amide, or ester thereof at a frequency of once weekly or once every two weeks; and/or
after administering the second' dose for about 2, about 3, about 4, about 5, about 6, about 7, or about 8 weeks, administering a third' dose of about 3-7mg of the compound of formula (I), or the pharmaceutically acceptable salt, amide, or ester thereof at a frequency of once weekly or once every two weeks; and/or
after administering the third' dose for about 2, about 3, about 4, about 5, about 6, about 7, or about 8 weeks, administering a fourth' dose of about 5-9mg of the compound of formula (I), or the pharmaceutically acceptable salt, amide, or ester thereof at a frequency of once weekly or once every two weeks; and/or
after administering the fourth' dose for about 2, about 3, about 4, about 5, about 6, about 7, about 8, about 9, about 10, about 11, about 12, about 13, about 14, about 15, about 16, about 17, about 18, about 19, or about 20 weeks, administering a fifth' dose of about 7-12mg of the compound of formula (I), or the pharmaceutically acceptable salt, amide, or ester thereof at a frequency of once weekly or once every two weeks; and/or after administering the fifth' dose for about 2, about 3, about 4, about 5, about 6, about 7, about 8, about 9, about 10, about 11, about 12, about 13, about 14, about 15, about 16, about 17, about 18, about 19, or about 20 weeks, administering a sixth' dose of about 9-15mg of the compound of formula (I), or the pharmaceutically acceptable salt, amide, or ester thereof at a frequency of once weekly or once every two weeks; and/or
after administering the sixth' dose for about 2, about 3, about 4, about 5, about 6, about 7, about 8, about 9, about 10, about 11, about 12, about 13, about 14, about 15, about 16, about 17, about 18, about 19, or about 20 weeks, administering a seventh' dose of about 12-18mg of the compound of formula (I), or the pharmaceutically acceptable salt, amide, or ester thereof at a frequency of once weekly or once every two weeks; and/or
after administering the seventh' dose for about 2, about 3, about 4, about 5, about 6, about 7, about 8, about 9, about 10, about 11, about 12, about 13, about 14, about 15, about 16, about 17, about 18, about 19, or about 20 weeks, administering an eighth' dose of about 15-24mg of the compound of formula (I), or the pharmaceutically acceptable salt, amide, or ester thereof at a frequency of once weekly or once every two weeks; and/or
after administering the eighth' dose for about 2, about 3, about 4, about 5, about 6, about 7, about 8, about 9, about 10, about 11, about 12, about 13, about 14, about 15, about 16, about 17, about 18, about 19, or about 20 weeks, administering a ninth' dose of about 18-30mg of the compound of formula (I), or the pharmaceutically acceptable salt, amide, or ester thereof at a frequency of once weekly or once every two weeks; and/or
after administering the ninth' dose for about 2, about 3, about 4, about 5, about 6, about 7, about 8, about 9, about 10, about 11, about 12, about 13, about 14, about 15, about 16, about 17, about 18, about 19, or about 20 weeks, administering a tenth' dose of about 24-48mg of the compound of formula (I), or the pharmaceutically acceptable salt, amide, or ester thereof at a frequency of once weekly or once every two weeks.

22. The method according to any one of claims 1-6, 7-9 or 21, wherein the first' dose is about 0.5-3mg, preferably about 0.5mg, about 1mg, about 1.5mg, about 2mg, about 2.5mg, or about 3mg, preferably about 1.5mg; and/or
the second' dose is about 1-5mg, preferably about 1mg, about 2mg, about 3mg, about 4mg, or about 5mg, preferably about 3mg; and/or
the third' dose is about 3-7mg, preferably about 3mg, about 4mg, about 5mg, about 6mg, or about 7mg, preferably about 5mg; and/or
the fourth' dose is about 5-9mg, preferably about 5mg, about 6mg, about 7mg, about 8mg, about 9mg, preferably about 6mg or about 7mg; and/or
the fifth' dose is about 7-12mg, preferably about 7mg, about 8mg, about 9mg, about 10mg, about 11mg, or about 12mg, preferably about 9mg; and/or
the sixth' dose is about 9-15mg, preferably about 9mg, about 10mg, about 11mg, about 12mg, about 13mg, about 14mg, or about 15mg, preferably about 12mg; and/or
the seventh' dose is about 12-18mg, preferably about 12mg, about 13mg, about 14mg, about 15mg, about 16mg, about 17mg, or about 18mg, preferably about 15mg; and/or
the eighth' dose is about 15-24mg, preferably about 15mg, about 16mg, about 17mg, about 18mg, about 19mg, about 20mg, about 21mg, about 22mg, about 23mg, or about 24mg, preferably about 18mg; and/or
the ninth' dose is about 18-30mg, preferably about 18mg, about 19mg, about 20mg, about 21mg, about 22mg, about 23mg, about 24mg, about 25mg, about 26mg, about 27mg, about 28mg, about 29mg, about 30mg, preferably about 24mg; and/or
the tenth' dose is about 24-48mg, preferably about 24mg, about 25mg, about 26mg, about 27mg, about 28mg, about 29mg, about 30mg, about 31mg, about 32mg, about 33mg, about 34mg, about 35mg, about 36mg, about 37mg, about 38mg, about 39mg, about 40mg, about 41mg, about 42mg, about 43mg, about 44mg, about 45mg, about 46mg, about 47mg, or about 48mg, preferably about 30mg.

23. The method according to any one of claims 1-6, 7-9 or 21-22, wherein the method comprises administering to a subject in need thereof a first' dose of about 1.5mg of the compound of formula (I), or the pharmaceutically acceptable salt, amide or ester thereof at a frequency of once weekly or once every two weeks;
after administering the first' dose for about 1 week, administering a second' dose of about 3mg of the compound of formula (I), or the pharmaceutically acceptable salt, amide or ester thereof at a frequency of once weekly;
after administering the second' dose for about 2 weeks, administering a third' dose of about 5mg of the compound of formula (I), or the pharmaceutically acceptable salt, amide or ester thereof at a frequency of once weekly;
after administering the third' dose for about 4 weeks, administering a fourth' dose of about 7mg of the compound of formula (I), or the pharmaceutically acceptable salt, amide or ester thereof at a frequency of once weekly;
after administering the fourth' dose for about 4 weeks, administering a fifth' dose of about 9mg of the compound of formula (I), or the pharmaceutically acceptable salt, amide or ester thereof at a frequency of once weekly or once every two weeks;
after administering the fifth' dose for about 4 weeks, administering a sixth' dose of about 12mg of the compound of formula (I), or the pharmaceutically acceptable salt, amide or ester thereof at a frequency of once weekly or once every two weeks;
after administering the sixth' dose for about 4 weeks, administering a seventh' dose of about 15mg of the compound of formula (I), or the pharmaceutically acceptable salt, amide or ester thereof at a frequency of once weekly or once every two weeks;
after administering the seventh' dose for about 4 weeks, administering a eighth' dose of about 18mg of the compound of formula (I), or the pharmaceutically acceptable salt, amide or ester thereof at a frequency of once weekly or once every two weeks;
after administering the eighth' dose for about 4 weeks, administering a ninth' dose of about 24mg of the compound of formula (I), or the pharmaceutically acceptable salt, amide or ester thereof at a frequency of once weekly or once every two weeks; and
after administering the ninth' dose for about 4 weeks, administering a tenth' dose of about 30mg of the compound of formula (I), or the pharmaceutically acceptable salt, amide or ester thereof at a frequency of once weekly or once every two weeks, preferably administering the tenth' dose of about 30mg of the compound of formula (I), or the pharmaceutically acceptable salt, amide or ester thereof at the frequency of once weekly or once every two weeks for 2 weeks or more, preferably 4 weeks or more, preferably 8 weeks or more, preferably 12 weeks or more, preferably 16 weeks or more, preferably 20 weeks or more; preferably administering the tenth' dose of about 30mg of the compound of formula (I), or the pharmaceutically acceptable salt, amide or ester thereof at a frequency of once weekly or once every two weeks for about 4 weeks.

24. The method according to any one of claims 1-23, **characterized in that**, the compound of formula (I), or the pharmaceutically acceptable salt, amide or ester thereof, is administered parenterally, preferably subcutaneously.

25. The method according to any one of claims 1-24, **characterized in that**, the compound of formula (I), or the pharmaceutically acceptable salt, amide or ester thereof is administered for 10 weeks or more, preferably 15 weeks or more, preferably 20 weeks or more, preferably 25 weeks or more, preferably 30 weeks or more, preferably 35 weeks or more, preferably 12 months or more, preferably 16 months or more, preferably 18 months or more, preferably 24 months or more, preferably 28 months or more, preferably 29 months or more, preferably 30 months or more.

26. The method according to any one of claims 1-25, **characterized in that**, a subject has a BMI ≥ 24kg/m², preferably BMI ≥ 25kg/m², preferably BMI ≥ 26kg/m²;
preferably:
the subject is accompanied by at least one of the following manifestations:
1) comorbidity with one or more of prediabetes, hypertension, dyslipidemia, or fatty liver;
2) comorbidity with weight-bearing joint pain; and
3) Obesity-induced dyspnoea or obstructive sleep apnea syndrome.

27. The method according to any one of claims 1-26, the compound of formula (I), or the pharmaceutically acceptable salt, amide or ester thereof, is administered in the form of a pharmaceutical composition comprising one or more pharmaceutically acceptable excipients.

28. The method according to claim 27, the pharmaceutical composition comprises:
about 3mg/ml, about 6mg/ml, about 12mg/ml, about 18mg/ml, about 24mg/ml, about 36mg/ml, or about 48mg/ml of the compound of formula (I), or the pharmaceutically acceptable salt, amide, or ester thereof;
about 5mg/ml, about 6mg/ml, about 7mg/ml, about 8mg/ml, about 9mg/ml or about 10mg/ml of NaCl, preferably about 7mg/ml of NaCl;
about 1mM, 2mM, 3mM, 4mM, 5mM, 6mM, 7mM, 8mM, 9mM or 10mM of citric acid;
about 1.42 mg/ml, about 1.5 mg/ml, about 2 mg/ml, about 2.5 mg/ml, about 3 mg/ml, about 3.5 mg/ml, about 4 mg/ml, or about 4.5 mg/ml of a Na₂HPO₄ solution, preferably about 1.42 mg/ml of Na₂HPO₄; and
the pH of the pharmaceutical composition is about 7.2, about 7.3, about 7.4, about 7.5, about 7.6, about 7.7, about 7.8, about 7.9, about 8.0, about 8.1, or about 8.2, preferably about 7.3.

29. The method according to any one of claims 1-28, **characterized in that**, after administration of the compound of formula (I) or the pharmaceutically acceptable salt, amide, or ester thereof, a subject achieves a weight loss of about 5% or more, preferably about 10%-60%, preferably about 10%-50%, preferably about 10%-40%, preferably about 10%-30%, preferably about 10%-25%, preferably about 10%, about 10.16%, about 11%, about 11.15%, about 12%, about 12.23%, about 13%, about 13.22%, about 13.26%, about 13.5%, about 14%, about 14.25%, about 15%, about 16%, about 16.3%, about 16.79%, about 17%, about 17.29%, about 17.78%, about 18%, about 18.6%, about 19%, or about 20%; preferably, after administering the compound of formula (I) or the pharmaceutically acceptable salt, amide, or ester thereof for about 10 weeks, about 20 weeks, about 25 weeks, about 30 weeks or about 35 weeks, the subject achieves a weight loss of about 10% or more, preferably about 10%-30%, preferably about 10%-25%, preferably about 10%, about 10.16%, about 11%, about 11.15%, about 12%, about 12.23%, about 13%, about 13.22%, about 13.26%, about 13.5%, about 14%, about 14.25%, about 15%, about 16%, about 16.3%, about 16.79%, about 17%, about 17.29%, about 17.78%, about 18%, about 18.6%, about 19%, or about 20%.

30. The method according to any one of claims 1-29, **characterized in that**, after administration of the compound of formula (I) or the pharmaceutically acceptable salt, amide, or ester thereof, a subject achieves a weight loss of about 10% or more, preferably about 10%-30%, preferably about 10%-25%, preferably about 10%, about 11%, about 12%, about 13%, about 13.5%, about 14%, about 15%, about 16%, about 17%, about 18%, about 18.6%, about 19%, or about 20%; preferably, after administering the compound of formula (I) or the pharmaceutically acceptable salt, amide, or ester thereof for about 10 weeks, about 20 weeks, about 25 weeks, about 30 weeks or about 35 weeks, a subject achieves a weight loss of about 10% or more, preferably about 10%-30%, preferably about 10%-25%, preferably about 10%, about 11%, about 12%, about 13%, about 13.5%, about 14%, about 15%, about 16%, about 17%, about 18%, about 18.6%, about 19%, or about 20%.

31. The method according to claim 15, **characterized in that**, when the fourth dose of about 12mg of the compound of formula (I), or the pharmaceutically acceptable salt, amide or ester thereof, is administered at the frequency of every two weeks for 20 weeks or more, the average body weight of the subject is reduced by about 11.15% or more compared to baseline.

32. The method according to claim 16, **characterized in that**, when the fifth dose of about 18mg of the compound of formula (I), or the pharmaceutically acceptable salt, amide or ester thereof, is administered at the frequency of every two weeks for 16 weeks or more, the average body weight of the subject is reduced by about 13.22% or more compared to baseline.

33. The method according to claim 17, **characterized in that**, when the sixth dose of about 24mg of the compound of formula (I), or the pharmaceutically acceptable salt, amide or ester thereof, is administered at the frequency of every two weeks for 12 weeks or more, the average body weight of the subject is reduced by about 14.25% or more compared to baseline.

34. The method according to claim 18, **characterized in that**, when the seventh dose of about 48mg of the compound of formula (I), or the pharmaceutically acceptable salt, amide or ester thereof, is administered at the frequency of every two weeks for 8 weeks or more, the average body weight of the subject is reduced by about 17.29% or more compared to baseline.

35. The method according to claim 17, **characterized in that**, when the sixth dose of about 24mg of the compound of formula (I), or the pharmaceutically acceptable salt, amide or ester thereof, is administered at the frequency of once weekly for 12 weeks or more, the average body weight of the subject is reduced by about 17.78% or more compared to baseline.
